(19) Europäisches Patentamt European Patent Office Office européen des brevets

(11) **EP 3 812 772 B1**

(12) **EUROPEAN PATENT SPECIFICATION**

(45) Date of publication and mention of the grant of the patent:
**19.02.2025 Bulletin 2025/08**

(21) Application number: **20174840.7**

(22) Date of filing: **14.05.2020**

(51) International Patent Classification (IPC):
*G01N 33/68* (2006.01)    *G01N 33/86* (2006.01)

(52) Cooperative Patent Classification (CPC):
**G01N 33/86; G01N 33/6893;** G01N 2333/75;
G01N 2800/224; G01N 2800/26

(54) **METHOD FOR DIAGNOSING FIBRINOLYTIC INSUFFICIENCY RELATED TO NEUTROPHIL EXTRACELLULAR TRAPS**

VERFAHREN ZUR DIAGNOSE VON FIBRINOLYTISCHER INSUFFIZIENZ IM ZUSAMMENHANG MIT NEUTROPHILEN EXTRAZELLULÄREN FALLEN

PROCÉDÉ DE DIAGNOSTIC D'UNE INSUFFISANCE FIBRINOLYTIQUE LIÉE À DES PIÈGES EXTRACELLULAIRES NEUTROPHILES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2019 EP 19306378**

(43) Date of publication of application:
**28.04.2021 Bulletin 2021/17**

(73) Proprietors:
• **Hôpitaux Universitaires de Strasbourg (HUS)**
**67000 Strasbourg (FR)**
• **INSERM - Institut National de la Santé et de la Recherche Médicale**
**75013 Paris (FR)**

(72) Inventors:
• **MEZIANI, Ferhat**
**67205 Oberhausbergen (FR)**
• **ANGLES-CANO, Eduardo**
**75013 Paris (FR)**
• **TOTI, Florence**
**67400 Illkirch (FR)**

(74) Representative: **Novagraaf Technologies Bâtiment O2**
**2, rue Sarah Bernhardt**
**CS90017**
**92665 Asnières-sur-Seine Cedex (FR)**

(56) References cited:
**WO-A2-2017/027611    CN-A- 1 896 232**

• **DAYANA BARBOSA DA CRUZ ET AL: "DNA-bound elastase of neutrophil extracellular traps degrades plasminogen, reduces plasmin formation, and decreases fibrinolysis: proof of concept in septic shock plasma", THE FASEB JOURNAL, vol. 33, no. 12, 4 November 2019 (2019-11-04), US, pages 14270 - 14280, XP055738675, ISSN: 0892-6638, DOI: 10.1096/ fj.201901363RRR**

## Description

### Technical field

The present invention relates to an in-vitro process for predicting and/or detecting fibrinolytic insufficiency, associated with NETs with/without disseminated intravascular coagulation (DIC), from a first biological sample comprising measuring the concentration of plasminogen and/or at least one fragment thereof. The present invention also relates to a process for determining the efficiency of a treatment of fibrinolytic insufficiency.

The present invention is applicable especially in the medical and veterinary field.

In the description below, the references between brackets ( ) refer to the reference list at the end of the text.

### Prior art

Septic shock is a syndrome characterized by a dysregulated inflammatory reaction of the host in response to a pathogenic agent, inducing hemodynamic dysfunction, and coagulation-fibrinolytic disorders leading to multiple organ failure syndrome.

Thrombus lysis, a major line of defense against thrombosis, is triggered by plasmin generated upon cleavage-activation of plasminogen bound to clot components, mostly fibrin or platelet membranes.(1) Plasminogen is a single-chain molecule consisting of an N-terminal region, 5 kringle (K) domains and the serine protease (SP) region.(2) $K_1$ and $K_4$ contain each a lysine-binding site that ensures binding of plasminogen to carboxy-terminal lysine residues of fibrin.(3) Plasminogen thus bound is cleaved at $Arg_{561}$-$Val_{562}$ and transformed into plasmin by the tissue-type or the urokinase-type plasminogen activators, tPA and uPA respectively, in a surface and localized dependent manner. Purposely, tPA is fully active once bound to fibrin whereas uPA preferentially operates when bound to its membrane-anchored receptor uPAR. Proteases like human neutrophil elastase (HNE) degrade plasminogen without generating plasmin (4, 5). In the circulation, HNE is rapidly inhibited by the $\alpha_1$-proteinase inhibitor, ($\alpha_1$-PI, Ka = $6.5 \times 10^7$ M$^{-1}$ s$^{-1}$)(6) that circulates at a high concentration (20 to 40 $\mu$M). HNE-$\alpha_1$PI complexes thus formed preclude proteolytic degradation of circulating plasminogen. However, experimental evidence indicates that HNE bound to negatively charged membrane proteoglycans or other macromolecules escapes inhibition and remains active.(7, 8) Belorgey and Bieth (1995, 1998)(9, 10) demonstrated that HNE forms a tight complex with DNA that strongly impairs its inhibition by $\alpha_1$-PI. Besides this protective action on HNE, the group of Bieth fully documented the protective action of DNA on other bound proteases.(11, 12) The extrusion into the extracellular space of functional scaffolds combining intact DNA with added-on HNE by neutrophils in a process termed neutrophil extracellular traps (NETs) formation or netosis, was discovered in 2004.(13) PMA (phorbol 12-myristate 13-acetate) and bacterial infections use a related pathway for NET induction: activation of protein kinase C and generation of reactive oxygen species.(14)

NETs are decondensed nuclear DNA fibers with bound nuclear proteins (histones), and cytoplasmic and granular proteases including HNE, cathepsin G and myeloperoxidase.(15) HNE is the most abundant (5.24 $\mu$moles per g NET-DNA),(16) characteristic component of NETs that is bound to DNA with nanomolar affinity.(17) NETs are proteolytically active and were originally described as beneficial (16) extracellular killers of microorganisms.(18) Recent data indicate that NETs are also of pathophysiological relevance in a variety of non-infectious inflammatory disorders, including autoimmune diseases, thrombosis (19, 20) and cancer (74) The combined action of NETs and the clotting system characterizes immunothrombosis.(20) It has been shown that NETs are released during thrombus formation and are integrated together with von Willebrand factor into the fibrin scaffold.(21, 22) The presence of NETs in human coronary and cerebral thrombi has actually been demonstrated (23-25). The NETs' DNA-backbone insensitivity to plasmin and the formation of non-fibrinolytic plasmin-DNA-fibrin complexes were suggested as possible causes of fibrinolytic resistance (27, 29) favoring thrombosis.

However, there is currently no process that makes it possible to safely and systematically detect fibrinolytic insufficiency, in particular associated to netosis, in particular in infectious and non-infectious diseases. There is also no process and/or means that makes it possible to predict the occurrence, for example from a biological sample, of fibrinolytic insufficiency, in particular associated to netosis and/or DIC.

Some possible biological markers of DIC and secondary fibrinolytic response have been disclosed.

However, the known markers may be detected/used in particular conditions and/or by using complex device and/or method and/or allow to obtain results after a delay which is too long or could be too long to be useful to the patient and/or the clinician and/or doctor.

There is therefore a real need to find a process and/or means that overcomes these flaws, drawbacks and obstacles of the prior art, in particular a simple, quick and effective process making it possible to detect fibrinolytic insufficiency and fibrinolytic insufficiency associated to netosis and DIC, in order to improve the vital prognosis of individuals.

There is also a real need to find a process and/or means that overcomes these flaws, drawbacks and obstacles of the prior art, in particular a simple, quick and effective process making it possible to detect fibrinolytic insufficiency and fibrinolytic insufficiency associated to netosis and DIC, in order to improve the vital prognosis of individuals and/or reduce treatment costs for said individuals.

**Description of the invention**

The invention is defined in the appended claims.

The aim of the present invention is to overcome the drawbacks of the prior art by providing a process for predicting and/or detecting fibrinolytic insufficiency, preferably fibrinolytic insufficiency associated to netosis and/or disseminated intravascular coagulation (DIC) from a biological sample comprising a step of detecting and/or measuring the concentration and/or content of at least one biological marker.

In the present document, "biological marker" is intended to mean at least one biological marker selected from the group comprising plasminogen or fragment thereof, and/or a protease component of NETs, preferably Human Neutrophil Elastase (HNE). Preferably the biological marker is plasminogen or fragment thereof, more preferably plasminogen and/or a protease-derived plasminogen fragment, more preferably HNE-derived plasminogen fragment.

According to the invention, the content of said at least one selected biological marker, in particular in a sample, may be measured by any suitable process known to those skilled in the art.

According to the invention, the concentration of said at least one selected biological marker, in particular in a sample, may be measured by any suitable process known to those skilled in the art.

According to the invention, said at least one selected biological marker may be detected by any suitable process known to those skilled in the art.

Since NETs may compromise thrombus lysis, the inventors investigate the role of HNE-DNA complexes on fibrinolysis. The inventors surprisingly demonstrate that in the presence of NETs containing active HNE-DNA complexes, plasminogen is reduced to fragments that inhibit plasminogen binding and plasmin formation onto fibrin resulting in impaired fibrinolysis. The inventors surprisingly demonstrate new mechanistic insights into septic shock thrombosis based on the capacity of HNE-DNA complexes to trigger a new unconventional mechanism of fibrinolytic insufficiency.

The inventors also surprisingly demonstrate that the concentration of Plasminogen is reduced in biological sample from a patient with septic shock.

The inventors also surprisingly demonstrate that the concentration of Plasminogen is reduced in biological sample from a patient with fibrinolytic insufficiency, in particular with fibrinolytic insufficiency associated to netosis and disseminated intravascular coagulation (DIC).

The inventors also surprisingly demonstrate in patients suffering from septic shock induced DIC and NETosis, plasminogen degradation by human neutrophil elastase (HNE) bound to DNA and subsequent failure to form plasmin.

The inventors have also surprisingly demonstrated that NETs induce a decrease of plasminogen concentration, in particular plasminogen concentration in the plasma, in particular in patients suffering from septic shock associated-disseminated intravascular coagulation (DIC).

An object of the present invention is an in-vitro process for predicting and/or detecting fibrinolytic insufficiency associated to NETs associated and disseminated intravascular coagulation (DIC) from a biological sample comprising measuring the concentration of Plasminogen and/or fragment thereof.

An object of the present invention is also an in-vitro process for predicting and/or detecting fibrinolytic insufficiency associated to pathologies with NETs with/without disseminated intravascular coagulation (DIC), from a first biological sample comprising measuring the concentration of Plasminogen and/or at least one fragment thereof.

An object of the present invention is also an in-vitro process for predicting and/or detecting pathology associated with NETs preferably sepsis, ischemic stroke, coronary thrombosis, cancer, and autoimmune diseases from a biological sample comprising measuring the concentration of plasminogen and/or fragments thereof, preferably mini-plasminogen (K5-SP).

In the present document, "netosis" is the process of neutrophil death whereas NETs refer specifically to DNA fibers with bound nuclear proteins and active enzymes, preferentially DNA-HNE complexes.

In the present document "protease component of NETs" means actives enzymes bound to DNA fibers of NETs.

In the present document "a protease-derived plasminogen fragment" means any plasminogen fragments known from on skilled in the art.

In the present document "fibrinolytic insufficiency" means any disorder and/or deficiency in the fibrinolytic process. It may be for example any deficiency that could involve fibrinolytic factors, for example plasminogen, tissue plasminogen activator [tPA], urokinase [uPA], and the inhibitors PAI-1 and a2-antiplasmin. Preferably it means fibrinolytic insufficiency, associated with neutrophil extracellular traps (NET) release, and/or with disseminated intravascular coagulation (DIC).

In the present document "disseminated intravascular coagulation" (DIC) means a condition characterized by systemic activation of coagulation, potentially leading to thrombotic obstruction of small and midsize vessels, thereby contributing to organ dysfunction, as disclosed in Blood.

In the present document "pathology associated with NETs or Netosis" means any pathology known from one skilled in the art which involved NETs or Netosis. It may be for example sepsis, ischemic stroke, coronary thrombosis, cancer, and/or autoimmune diseases. In particular it may be NETs associated with sepsis-DIC, NETs associated with acute myocardial infarction or stent thrombosis, NETs associated with ischemic stroke thrombi, NETs associated with autoimmune diseases such as systemic lupus erythematosus and rheumatoid arthritis or NETs associated with breast or lung or pancreatic cancer.

In the present document "plasminogen" means a 92-kDa protein which is a single-chain molecule consisting of an N-terminal region, 5 kringle (K) domains ($K_1$ to $K_5$) and the serine protease (SP) region. (2) $K_1$ and $K_4$ contain each a lysine-binding site. In other words, "Plasminogen" is intended to mean a 92 kDa single-chain proenzyme consisting of an N-terminal region, 5 kringle (K) domains and the serine protease (SP) region. It may be for example plasminogen as described by Xue Y et al (2) and/or by Law et al. Cell Reports| Volume 1, ISSUE 3, P185-190, March 29, 2012(75).

In the present "plasminogen fragments" means any fragments of plasminogen known from one skilled in the art. It may be for example fragments of plasminogen comprising any domain of plasminogen, for example of kringle (K) 1 to 5 domains or the serine protease domain (SP), or any combination thereof, for example a fragment composed of K1+K2+K3, a fragment composed of K1+K2+K3+K4, or a fragment composed of single K4 It may be for example a fragment composed of domains K5 and the SP also called mini-plasminogen (K5-SP) and /or any fragment of plasminogen obtained with active protease-DNA complexes preferably HNE-DNA complexes of NETs.

In the present "HNE-derived plasminogen fragments" means fragments of plasminogen selected from the group comprising kringle 1 to 3 domains ($K_{1+2+3}$), kringle 1 to 4 domains ($K_{1+2+3+4}$) and kringle 5 domain and the serine protease (SP) region, also designated mini-plasminogen, ($K_5$-SP).

In the present "cytoplasmic and granular active proteases" means cathepsin G, myeloperoxidase or elastase.

In the present "Human Neutrophil Elastase" (HNE) means Neutrophil elastase (EC 3.4.21.37), leukocyte elastase. HNE is a trypsin/chymotrypsin-type serine proteinase in the same family of others leukocyte granule-associated proteases, for example proteinase 3 and cathepsin.

In the present "DNA bound proteins" means histones, preferentially citrullinated histones, fibronectin and von Willebrand factor.

In the present document, "biological sample" is intended to mean any sample obtained from mammals, for example a mammal selected from the group comprising the orders Monotremata, Didelphimorphia, Paucituberculata, Microbiotheria, Notoryctemorphia, Dasyuromorphia, Peramelemorphia, Diprotodontia, Tubulidentata, Sirenia, Afrosoricida, Macroscelidea, Hyracoidea, Proboscidea, Cingulata, for example the armadillo, Pilosa, Scandentia, Dermoptera, Primates, Rodentia, Lagomorpha, Erinaceomorpha for example the hedgehog, Soricomorpha, Chiroptera, Pholidota, Carnivora, Perissodactyla, Artiodactyla and Cetacea. It may for example be a human or an animal. It may for example be a livestock animal, a pet, a threatened animal species or any other animal.

According to the invention, the biological sample may be a blood sample, plasma sample or any biological fluid. It may be for example a biological sample selected from the group comprising blood, plasma, urine, tears or tissue extracts. Preferably, the biological sample may be a blood sample or a plasma sample, more preferably plasma sample, it may also be a culture supernatant obtained from cultured cells in vitro.

According to the invention, the biological sample may originate from a mammal with septic shock or a risk of septic shock or any other non-infectious inflammatory process.

According to the invention, the concentration of plasminogen may be determined/measured by any method and/or process known form one skilled in the art. It may be, for example a concentration measured by antigenic or by a functional assay using either a plasminogen activator e.g. urokinase, or streptokinase-plasminogen complexes, for example as disclosed in "Criteria for specific measurement of plasminogen (enzymatic; procedure) in human plasma", eJIFCC, vol 12 No 2, 2000 (77) or in Massberg, S., et al. "Reciprocal coupling of coagulation and innate immunity via neutrophil serine proteases". Nat Med 16, 887-896 (45). The concentration of plasminogen may also be determined/measured with a process comprising measuring its potential fibrinolytic activity, by immunoassay, by cellular immunoassay, or flow cytometry.

For example, when the concentration of plasminogen is measured by immunoassay, the immunoassay allow to detect circulating plasminogen, when the concentration of plasminogen is measured by cellular immunoassay, the cellular immunoassay may use flow cytometry to detect plasminogen on cell surface. It may be for example an immunoassay as disclosed in Rife U, Milgrom F, Shulman S. Antigenic analysis of plasminogen and plasmin. Blood 1963,212,322 Okamoto A et al. Population based distribution of plasminogen... J Thromb Haemost 2003,1,2397 (83).

For example when the concentration of plasminogen is measured by flow it may allow to determine plasminogen binding to identify cell surface-specific plasminogen binding

For example, the concentration of plasminogen may be measured by a colorimetric method, for example colorimetric method using streptokinase as activator, for example using a commercial available kit, for example BIOPHEN Plasminogen (LRT) Ref 221511 commercialized by Hyphen Biomed, for example COAMATIC® Plasminogen Ref 82 2452 63 by Chromogenix ; for example Stachrom Plasminogen 00658 by STAGO. For example, when the concentration of plasminogen is measured with BIOPHEN Plasminogen (LRT) Ref 221511, the reference or normal range is from 70 to 130%. For example, when the concentration of plasminogen is measured with COAMATIC® Plasminogen Ref 82 2452 63, the reference or normal range is from 80 to 120%. For example, when the concentration of plasminogen is measured with Stachrom Plasminogen 00658, the reference or normal range is from 80 to 120%.

For example, the concentration of plasminogen may be measured by immunoassay, for example using a commercial available kit, for example commercial kits available measure plasminogen by immunoassay, for example Technozym® Glu-Plasminogen ELISA Kit Ref TC12040 by Technoclone (Normal Glu-plasminogen values range from 60-250 $\mu$g/ml). For example the Human Plasminogen ELISA Kit (PLG) Ref ab108893 by Abcam. For example, when the concentration of plasminogen is measured with Human Plasminogen ELISA Kit (PLG), the reference or normal range of concentration of plasminogen in plasma is from 70 to 135 $\mu$g/mL.

For example, plasminogen may be measured/detected antigenic assay, for example by polyclonal and/or monoclonal antibodies, for example plasminogen Polyclonal and/or monoclonal antibodies commercially available, for example for flow cytometry to detect plasminogen on cell surface. It may be for example the antibodies commercialized with the reference PA5-14196 by Invitrogen and/or ab154560 by Abcam.

According to the invention, the concentration of fragments of plasminogen may be measured by any method and/or process known form one skilled in the art. It may be, for example a concentration measured by proteomic analysis using mass spectrophotometry or by antigenic assay using monoclonal antibodies or by any combination of methods. For example, the concentration of fragment of plasminogen comprising kringles 1-4 also called angiostatin-like may be measured by immunoassay, for example as disclosed by Lljnen et al. 2001 "Enzyme-linked Immunosorbent Assay for the Specific Detection of Angiostatin-Like Plasminogen Moieties in Biological Samples" Thromb Res 2001 102 (1), 53-9 (76). Plasminogen fragments including single K4, K1-K2-K3, K1-K2-K3-K4 or mini-plasminogen (K5-SP) may be preferably detected qualitatively by Western blot or Flow cytometry, for example using specific antibodies. It may be, for example a process as disclosed in M Sten-Linder et al. "Angiostatin Fragments in Urine From Patients With Malignant Disease" Anticancer Res 1999; 19 ;3409-14 (78), or in Y Takada "Potential Role of Kringle-Integrin Interaction in Plasmin and uPA" Actions Journal of Biomedicine and Biotechnology Volume 2012, Article ID 136302, 8 pages doi:10.1155/2012/136302 or in Z Zhang "Plasminogen Kringle 5 Inhibits Alkali-Burn-Induced Corneal Investigative" Ophthalmology & Visual Science November 2005, Vol.46, 4062-4071. doi:https://doi.org/10.1167/iovs.04-1330 - (80)

For example, when the concentration of fragment of plasminogen comprising kringles 1-4 also called angiostatin-like is measured by immunoassay, for example in tumor fluids from cancer patients, the process may comprise a removal step of other plasminogen moieties, for example by immunoadsorption, for example in samples, preferably in diluted sample.

According to the invention, the concentration of HNE-derived plasminogen fragments may be measured by any method and/or process known form one skilled in the art. It may be, for example a concentrations measured by antigenic assay using monoclonal antibodies. For example, the concentration of HNE-derived plasminogen fragments may be measured by immunoassay, for example as disclosed by Lljnen et al. 2001 (76). HNE-derived plasminogen fragments including single K4, K1-K2-K3, K1-K2-K3-K4 or mini-plasminogen (K5-SP) may be preferably detected qualitatively by Western blot or Flow cytometry, for example using specific antibodies. It may be, for example a process as disclosed in Rouy D et al. "Apolipoprotein(a) and Plasminogen Interactions With Fibrin: A Study With Recombinant Apolipoprotein(a) and Isolated Plasminogen Fragments" Biochemistry1992;31;6333 (56), or in Barbosa da Cruz D et al "DNA-bound Elastase of Neutrophil Extracellular Traps Degrades Plasminogen, Reduces Plasmin Formation, and Decreases Fibrinolysis: Proof of Concept in Septic Shock Plasma" FASEB J 20189,33,14270 (81). The concentration and quality of HNE fragments can also for example be measured and assessed by combining immunoantigenic methods and any other method of measurement of the size or determination of aminoacid sequence of the fragment, for example by mass spectrometry as for example those combined for proteomic analysis. For example, the concentration of HNE-derived plasminogen fragments may be measured by flow cytometry to detect binding of plasmin-generated protein fragments to cells., for example as disclosed in Constantinescu et al. "Amorphous protein aggregates stimulate plasminogen activation, leading to release of cytotoxic fragments that are clients for extracellular chaperones." J. Biol. Chem. (2017) 292(35) 14425-14437 (82).

For example, when the concentration of HNE-derived plasminogen fragments is measured by immunoassay, for example in tumor fluids from cancer patients, the process may comprise a removal step of other plasminogen moieties, for example by immunoadsorption, for example in samples, preferably in diluted sample.

According to the invention, the determination/measurement of the concentration of fragments of plasminogen comprising kringle (K) 1 to 3 domains ($K_{1+2+3}$) may be carried out by any adapted method known from one skilled in the art. It may be for example by antigenic assay. For example, the concentration of fragments of plasminogen comprising kringle (K) 1 to 3 domains ($K_{1+2+3}$) may be measured by immunoassay, for example as disclosed by Lijnen et al 2001 Enzyme-linked Immunosorbent Assay for the Specific Detection of Angiostatin-Like Plasminogen Moieties in Biological Samples Thromb Res 2001 102 (1), 53-9 (76).

According to the invention, the determination/measurement of the concentration of mini-plasminogen (K5-SP) or fragment of plasminogen comprising kringle (K) 5 domain and the serine protease (SP) region may be carried out by any adapted method known from one skilled in the art. It may be for example by antigenic assay. For example using a combination of protein separation and proteomic analysis by mass spectrometry. For example, the concentration of fragment of plasminogen comprising kringle (K) 5 domain and the serine protease (SP) region or mini-plasminogen (K5-SP) may be measured by immunoassay, for example as disclosed in Moroz et al. 1986 "Mini-plasminogen-likfer agments of plasminogen in synovial fluid in acute inflammatory arthritis Thromb Res 1986,43,417 (5).

According to the invention, the detection process may also comprise a step of comparing the measured concentration of plasminogen with a reference value. It may be for example a reference value or a reference intervals which represent usually of 95% of the value or interval of the reference healthy population, preferably determine through statistical analysis, for example as disclosed by The Clinical & Laboratory Standards Institute (CLSI).

According to the invention, the reference concentration value of plasminogen may be the concentration of plasminogen measured in a biological sample of subject, or the mean concentration value measured in a group of reference healthy subjects. Preferably, the reference concentration value of plasminogen may be the concentration of plasminogen measured in a pool of biological samples from healthy subjects. The reference concentration value of plasminogen may be internal standard defined as a biological sample of subject titrated against samples from healthy subjects. The reference concentration value of plasminogen may be for example a concentration of 1 to 2 $\mu$mol/L, preferably of 1.5 to 2 $\mu$mol/L of full-length plasminogen.

According to the invention, the reference concentration value of fragments of plasminogen may be the concentration of fragments of plasminogen measured a biological sample of subject, or the mean concentration value measured in a group of reference healthy subjects. Preferably, the reference concentration value of fragments of plasminogen may be the concentration of fragments plasminogen measured in a pool of biological samples from healthy subjects and/or in an internal standard defined as a biological sample of subject titrated against samples from healthy subjects.

According to the invention, the reference concentration value of fragments plasminogen comprising kringle (K) 1 to 3 domains ($K_{1+2+3}$), may be the concentration of fragment plasminogen comprising kringle (K) 1 to 3 domains ($K_{1+2+3}$) measured in a biological sample of subject, or the mean concentration value measured in a group of reference healthy subjects. Preferably the reference concentration value of fragments plasminogen comprising kringle (K) 1 to 3 domains ($K_{1+2+3}$), may be the concentration of fragment plasminogen comprising kringle (K) 1 to 3 domains ($K_{1+2+3}$) measured in a pool of biological samples from healthy subjects and/or in an internal standard defined as a biological sample of subject titrated against samples from healthy subjects.

According to the invention, the reference concentration value of fragment plasminogen comprising kringle (K) 5 domain and the serine protease (SP) region or mini-plasminogen, (K5-SP), may be the concentration of fragment plasminogen comprising kringle (K) 5 domain and the serine protease (SP) region or mini-plasminogen, (K5-SP) measured in a biological sample of subject, or the mean concentration value measured in a group of reference healthy subjects. Preferably the reference concentration value of fragment plasminogen comprising kringle (K) 5 domain and the serine protease (SP) region or mini-plasminogen, (K5-SP), may be the concentration of fragment plasminogen comprising kringle (K) 5 domain and the serine protease (SP) region or mini-plasminogen, (K5-SP) measured in a pool of biological samples from healthy subjects or in an internal standard defined as a biological sample of subject titrated against samples from healthy subjects.

In the present document, "reference healthy subject" or "healthy subject" is intended to mean a mammal, for example a human being, that has not been subject to an infection, shock, hemorrhage, septic shock and/or any other attack on the body capable of leading to fibrinolytic insufficiency and disseminated intravascular coagulation as defined above. It may be for example a human being, which has not been subject to an infectious or non-infectious pathology associated with NETs or Netosis, for example sepsis, ischemic stroke, coronary thrombosis, cancer, and autoimmune diseases as defined above.

According to the invention, "group of reference subjects" or "group of reference healthy subjects" is intended to mean a group making it possible to define a reliable reference value or reliable reference intervals. It may for example be a group comprising at least 2 reference subjects as defined above, for example at least 10, at least 40, at least 60, at least 100 reference subjects or healthy subjects. It may for example be a group comprising from 30 to 500 subjects, from 40 to 200, from 45 to 110 reference subjects or healthy subjects.

Advantageously, the inventors have demonstrated that when the measured concentration of plasminogen is lower than the reference value or intervals, the process according to the invention makes it possible to detect and/or predict disseminated intravascular coagulation with a fibrinolytic insufficiency.

Advantageously, the inventors have also demonstrated that when the content/concentration of plasminogen fragment is greater than the reference value or intervals, the process according to the invention makes it possible to detect and/or predict inhibition of fibrinolysis and/or fibrinolytic insufficiency, in particular fibrinolytic insufficiency in disseminated intravascular coagulation.

Advantageously, the inventors of the present invention have also demonstrated that the process for detecting fibrinolytic insufficiency comprising measuring the concentration of plasminogen and/or at least one fragment thereof enables detection of fibrinolytic insufficiency.

Advantageously, the inventors have demonstrated that when fragments of plasminogen, preferably mini-plasminogen (K5-SP) are detected in plasma in particular the process according to the invention makes it possible to detect and/or predict pathologies with NETs, for example sepsis, ischemic stroke, coronary thrombosis, cancer, and autoimmune diseases from a first biological sample or associated with Netosis.

Advantageously, the inventors of the present invention have also demonstrated that the process for detecting fibrinolytic insufficiency comprising measuring the concentration of plasminogen and/or at least one fragment thereof enables detection of pathology associated with NETs.

An object of the invention is also an in-vitro process for determining the efficacy of a treatment of fibrinolytic insufficiency associated associated to pathologies with NETs with/without disseminated intravascular coagulation (DIC), preferably sepsis, ischemic stroke, coronary thrombosis, cancer, and autoimmune diseases and/or pathology associated with Netosis comprising:

a. Determination and/or measurement of the concentration C1 of Plasminogen and/or fragment thereof from a first biological sample before treatment with a compound,
b. Determination and/or measurement of the concentration C2 of Plasminogen and/or fragment thereof from a first biological sample after treatment with said compound, and optionally
c. comparison of the concentrations and calculation of a score (S) according to the following formula:
S = C2/C1.

According to the invention, the biological sample is a biological sample as defined above.

According to the invention, the determination and/or measurement of the concentration of Plasminogen and/or fragment may be carried out by any method/process as defined/described above.

According to the invention plasminogen's fragment may be any plasminogen fragment as defined/described above.

According to the invention, when the process determines the efficacy of a treatment of fibrinolytic insufficiency and the biological marker is plasminogen, the treatment is effective when the value of S obtained from the comparison step is greater than 1.

According to the invention, when the process determines the efficacy of a treatment of fibrinolytic insufficiency and the biological marker is plasminogen fragment, the treatment is effective when the value of S obtained from the comparison step is lower than 1.

According to the invention, the in-vitro process for determining the efficacy of a treatment with a compound of fibrinolytic insufficiency associated associated to pathologies with NETs with/without disseminated intravascular coagulation (DIC), preferably sepsis, ischemic stroke, coronary thrombosis, cancer, and autoimmune diseases and/or pathology associated with Netosis comprising:

a. Determination and/or measurement of the concentration C1 of Plasminogen from a first biological sample before treatment with a compound,
b. Determination and/or measurement of the concentration C1F of Plasminogen's fragment from a second biological sample before treatment with a compound,
c. Determination and/or measurement of the concentration C2 of Plasminogen from a first biological sample after treatment with said compound,
d. Determination and/or measurement of the concentration C2F of Plasminogen's fragment from a second biological sample after treatment with said compound, and optionally
e. comparison of the concentrations and calculation of scores (S) and (SF) according to the following formula:

$$S = C2/C1,$$

$$SF = C2F/C1F$$

According to the invention, when the process determines the efficacy of a treatment of fibrinolytic insufficiency and the biological marker is plasminogen, the treatment is effective when the value of S obtained from the comparison step is greater than 1.

According to the invention, when the process determines the efficacy of a treatment of fibrinolytic insufficiency and the biological marker is plasminogen fragment, the treatment is effective when the value of SF obtained from the comparison step is lower than 1.

According to the invention, the first biological sample before treatment is a biological sample as defined above.

According to the invention, the second biological sample before treatment is a biological sample as defined above.

According to the invention, the second biological sample before treatment may be identical to or different from the first biological sample before treatment.

According to the invention, the first and second biological samples before treatment may be the same sample.

According to the invention, the first biological sample after treatment is a biological sample as defined above.

According to the invention, the second biological sample after treatment is a biological sample as defined above.

According to the invention, the second biological sample after treatment may be identical to or different from the first biological sample after treatment.

According to the invention, the first and second biological samples after treatment may be the same sample.

In the present "Treatment with a compound" means medical treatment, for example e.g. allopathic, involving the taking of molecules, e.g. chemical molecules, e.g. molecules obtained by organic synthesis, molecules of biological origin, e.g. proteins, molecules from and/or synthesized by living organisms, e.g. mammals, microorganisms, plants, or any other non-chemical treatment or any other device delivering the above mentioned molecules for example nanovesicles, engineered or not, for example conveying native plasminogen.

The inventors have surprisingly demonstrated that the supply of plasminogen to plasma samples of patients with septic shock-induced DIC allows to restore plasmin formation and fibrinolysis.

An object of the present disclosure, not part of the invention, is native and/or recombinant Plasminogen for use as a drug in the treatment of fibrinolytic insufficiency, in particular fibrinolytic insufficiency induced by low plasminogen concentration in patients with netosis and/or disseminated intravascular coagulation (DIC).

Also, an object of the present disclosure, not part of the invention, is a Pharmaceutical composition comprising Plasminogen for use as a medicament in the treatment of fibrinolytic insufficiency preferably associated to disseminated intravascular coagulation (DIC)

An object of the present disclosure, not part of the invention, is also a pharmaceutical composition comprising native and/or recombinant Plasminogen for use as a drug in the treatment of fibrinolytic insufficiency, preferably fibrinolytic insufficiency induced by low plasminogen concentration in patients with netosis and/or disseminated intravascular coagulation (DIC)

Another object of the present disclosure, not part of the invention, is Plasminogen, native and/or recombinant, for use as a drug in the treatment of disseminated intravascular coagulation (DIC), for example DIC linked to moderate/severe plasminogen consumption/deficiency, particularly in patients with septic shock induced DIC.

An object of the present disclosure, not part of the invention, is a pharmaceutical composition comprising plasminogen, native and/or recombinant, for use as a medicament in the treatment of disseminated intravascular coagulation (DIC), for example DIC linked to moderate/severe plasminogen consumption/deficiency, particularly in patients with septic shock and DIC.

In the present, the terms "treatment," "treat," "treated" or "treating" refer to prophylaxis and/or therapy, particularly wherein the object is to prevent or slow down (lessen) an undesired physiological change or disorder, such as the development and/or progression of disseminated intravascular coagulation. Beneficial or desired clinical results include, but are not limited to improve organs failure, stabilized (i.e., not worsening) state of disease, delay or slowing of disease progression, amelioration or palliation of the disease state, and healing (whether partial or total). "Treatment" can also mean prolonging survival and/or increased quality of life compared to expected survival and/or quality of life if not receiving treatment.

A "subject" or "patient" includes a mammal, e.g., a human, including a mammal in need of treatment for a disease or disorder, such as a mammal having been diagnosed with having a disease or disorder or determined to be at risk of developing a disease or disorder.

The pharmaceutical composition may be in any form that can be administered to a human or an animal.

The pharmaceutical composition may comprise plasminogen, for example a native and/or recombinant plasminogen. For example it may be Glu-plasminogen at a concentration of 6.6 mg/kg of subject.

Administration may be carried out directly, i.e. pure or substantially pure, or after mixing of plasminogen and/or fragment thereof with a pharmaceutically acceptable carrier and/or medium. According to the present invention, the pharmaceutical composition may be a syrup or an injectable solution. For example, when the pharmaceutical composition is an injectable solution it may be injected and/or administered as a 10- to 30-minute intravenous infusion.

According to the present disclosure, not part of the invention, the pharmaceutical composition comprising plasminogen may be administered every day, two day, three days... One skilled in the art, taking into consideration its technical knowledge would adapt the frequency of administration of the pharmaceutical composition comprising plasminogen.

According to the present disclosure, not part of the invention, when the fibrinolytic insufficiency is associated to netosis, the pharmaceutical composition may be tear drops or any preparation adapted to external application.

According to the present disclosure, not part of the invention, the pharmaceutical composition may be a pharmaceutical composition for oral administration selected from the group comprising a liquid formulation, an oral effervescent dosage form, an oral powder, a multiparticle system, an orodispersible dosage form. For example, when the pharmaceutical composition is for oral administration, it may be in the form of a liquid formulation selected from the group comprising a solution, a syrup, a suspension, an emulsion and oral drops. When the pharmaceutical composition is in the form of an oral effervescent dosage form, it may be in a form selected from the group comprising tablets, granules, and powders. When the pharmaceutical composition is the form of an oral powder or a multiparticulate system, it may be in a form selected from the group comprising beads, granules, mini tablets and micro granules. When the pharmaceutical composition is the form of an orodispersible dosage form, it may be in a form selected from the group comprising orodispersible tablets, lyophilized wafers, thin films, a chewable tablet, a tablet and a capsule, a medical chewing gum. According to the present invention, the pharmaceutical composition may be for buccal and sublingual routes, for example selected from the group comprising buccal or sublingual tablets, muco adhesive preparation, lozenges, oro-mucosal drops and sprays.

According to the present disclosure, not part of the invention, when the fibrinolytic insufficiency is associated to netosis, the pharmaceutical composition may be for topical-transdermal administration, for example selected from the group comprising ointments, cream, gel, lotion, patch and foam.

According to the present disclosure, not part of the invention, the pharmaceutical composition may be formulated for nasal administration, for example selected from the group comprising nasal drops, nasal spray, nasal powder. According to the present invention, the pharmaceutical may be formulated for rectal administration, for example suppository or hard gelatin capsule. According to the present invention, the pharmaceutical composition may be for parenteral administration, for example subcutaneous, intramuscular, intravenous administration. The skilled person in the art understands clearly that the term "form" as used herein refers to the pharmaceutical formulation for its practical use.

The pharmaceutically acceptable carrier may be any know pharmaceutically carrier used for the administration of native or recombinant plasminogen, depending on the subject. For example, pharmaceutically acceptable carrier, diluent or excipient includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent or emulsifier.

The form of the pharmaceutical composition may be selected with regards to the human or animal to be treated.

In another aspect, the present disclosure, not part of the invention, provides a method of treating a subject suffering from DIC, in particular DIC during septic shock. This method may comprise the step of administering to said subject native and/or recombinant plasminogen.

Native or recombinant plasminogen, as well as usable formulations are as defined above. The administration can be made by using any pharmaceutical way known by the skilled person and useful to administrate native and/or recombinant plasminogen, preferably the administration is a parenteral administration, preferably intravenous administration. Examples of administrable forms of medicament/drug are provided above.

Other advantages may become apparent to those skilled in the art on reading the examples below, illustrated by the appended figures, given by way of illustration.

**Brief description of the figures**

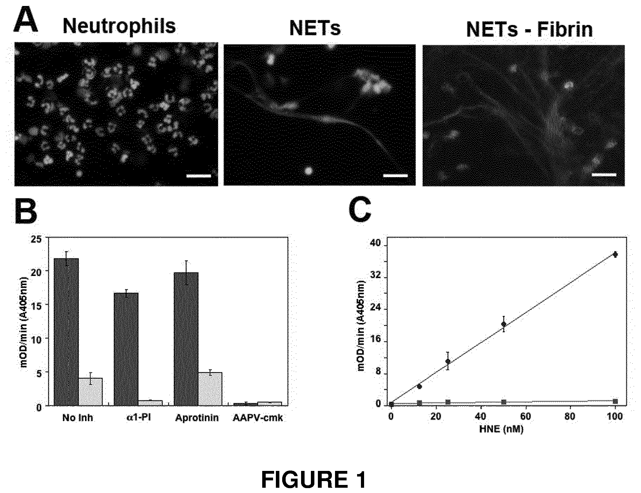

- Figure 1 represents Neutrophil Extracellular Traps (NETs) generated from neutrophils seeded onto a fibrin matrix: DNA and HNE activity of NETs are shown. Neutrophils isolated from human blood and seeded on wells of a 96-wells microtiter plate containing or not a fibrin matrix, were activated by 50 nM PMA in HBSS. After 4 hours incubation at 37°C in a humidified 5% $CO_2$ incubator, the plate was centrifuged at 3000 g for 5 min, the supernatant was collected and cells and NETs in the well stained with a 10$\mu$g/ml solution of Hoechst 33342 a minor-groove DNA binder. Elastase activity bound to NETs was detected with the chromogenic substrate N-methoxysuccinyl-Ala-Ala-Pro-Val-p-nitroanilide. Figure 1A represents fluorescence microscopy images (X20) of nuclei and extracellular DNA fibers stained with Hoechst. Scale bars: 20 $\mu$m. Neutrophils: non stimulated neutrophils incubated in the absence of PMA show typical polymorphonuclear appearance. NETs: neutrophil extracellular traps generated in the absence of fibrin. NETs-fibrin: NETs generated onto a fibrin matrix had a denser and reticulated appearance than NETs extruded in the absence of fibrin. Figure 1 B. represents the detection of HNE activity on HNE-ADN complexes of NETs (black bars) and in supernatants (grey bars) in the absence of inhibitor (No Inh) or in the presence of $\alpha$1-proteinase inhibitor ($\alpha$1-PI, 10 $\mu$M), Aprotinin (10 $\mu$M) or the synthetic inhibitor N-methoxysuccinyl-Ala-Ala-Pro-Val-chloromethyl ketone (AAPV-cmk, 100$\mu$M). C. Detection of soluble HNE activity in buffer (●) and its inhibition when added to normal human plasma (■). Purified HNE was incubated (15 min) at different concentrations in buffer or plasma before activity measurements with 1.5 mM of the chromogenic substrate N-methoxysuccinyl-Ala-Ala-Pro-Val-p-nitroanilide.

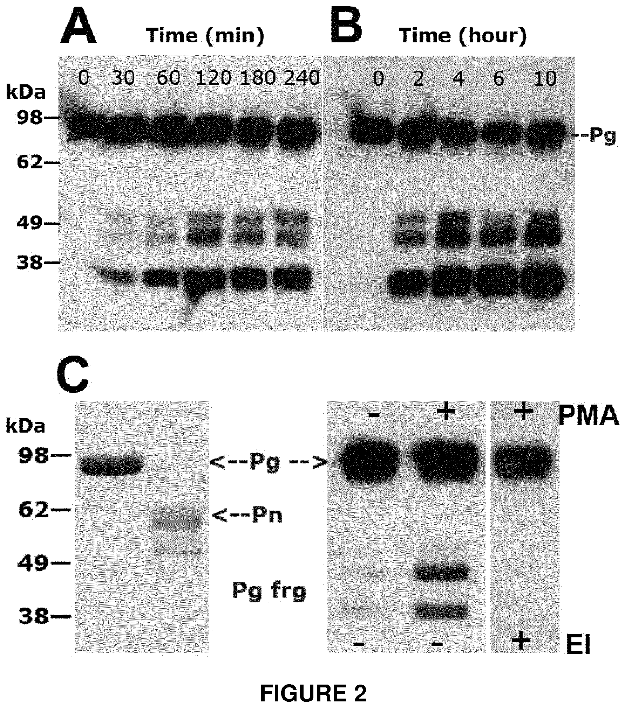

- **Figure 2** represents NETs-associated elastase digests plasminogen into fragments. Plasminogen (1 μM) was incubated with NETs generated by neutrophils treated with 50 nM PMA for 4 hours as indicated in figure 1. Samples were analyzed by SDS-PAGE and Western blot using a specific sheep antibody directed against human plasminogen. Figure 2A-B. Plasminogen fragmentation by NETs HNE•DNA complexes after 30 to 240 min (A) and 2 to 10 hours (B) of incubation. C. Western blot of purified plasminogen (Pg), plasmin (Pn) (left panel) and HNE-DNA-derived plasminogen fragments (middle panel). Plasminogen fragments produced by PMA stimulated neutrophils (middle panel) were not observed (right panel) in the presence of the elastase-specific inhibitor (EI) N-Methoxysuccinyl-Ala-Ala-Pro-Val-chloromethyl ketone.

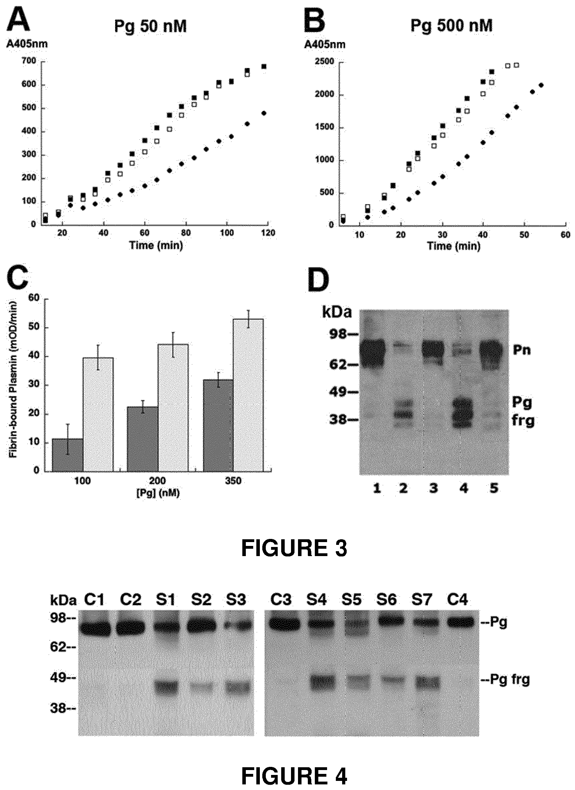

- **Figure 3** represents the decrease in tPA-mediated plasmin formation by NETs formed onto fibrin. Neutrophils isolated from human blood were seeded onto fibrin in a 96-wells microtitre plate and activated by 50 nM PMA in HBSS. NETs released by activated neutrophils were identified as indicated in figure 1. After binding of tPA to fibrin, plasminogen at varying concentrations was added to the interlaced NETs-fibrin structure. Figure 3 A-B. Plasmin formation from plasminogen (Pg) at 50 nM (A) and 500 nM (B) was monitored by measuring the released of pNA the plasmin-selective chromogenic substrate at 0.75 mM. □ Plasmin formation on fibrin. • Plasmin formation on fibrin-NETs. ■ Plasmin formation on fibrin-NETs treated with the elastase inhibitor MeO-Suc-AAPV-CMK. Figure 3 C. After plasminogen activation the wells were carefully washed and the amount of fibrin-bound plasmin detected by adding 0.75 mM of the plasmin-selective chromogenic substrate. Grey bars: plasmin bound to the fibrin-NETs surface. White bars: plasmin bound to the fibrin-NETs surface treated with the elastase inhibitor MeO-Suc-AAPV-CMK. Figure 3 D. Analysis of plasminogen fragmentation in protein extracts recovered from the fibrin-NETs surface after 2 hours of incubation. Western blot of non-reduced samples using a plasminogen-specific monoclonal antibody. 1: plasminogen activated by fibrin-bound tPA in the absence of NETs (in non-reduced gels plasminogen and plasmin migrates at a similar position). 2 and 4: Plasminogen incubated with the fibrin-NETs lattice; generation of plasminogen fragments and traces of plasmin. 3, 5. The generation of plasmin was recovered and the formation of plasminogen fragments inhibited in the presence of the elastase inhibitor MeO-Suc-AAPV-CMK.

- **Figure 4** represents plasminogen fragments circulating in patients with septic shock-induced disseminated intravascular coagulation. Plasma samples from patients and controls were diluted 1:5 in 125mM Tris-HCl buffer pH 6.8 containing 20% glycerol and 4% SDS. Proteins were separated by SDS-PAGE (10%) followed by Western blot using a specific sheep antibody directed against human plasminogen or the horseradish peroxidase-labelled monoclonal antibody CPL15-PO directed against kringle 1. C1-C4: Plasma from healthy donors. S1-S7: Samples from patients with septic shock-induced disseminated intravascular coagulation. Pg: plasminogen in samples from patients and healthy donors. Bands between 49 kDa and 38 kDa in S1-S7 represent plasminogen fragments (Pg frg). Plasma samples treated to eliminate high-abundant proteins by euglobulin precipitation or lysine-affinity fractionation were used for proteomic analysis of plasminogen fragments.

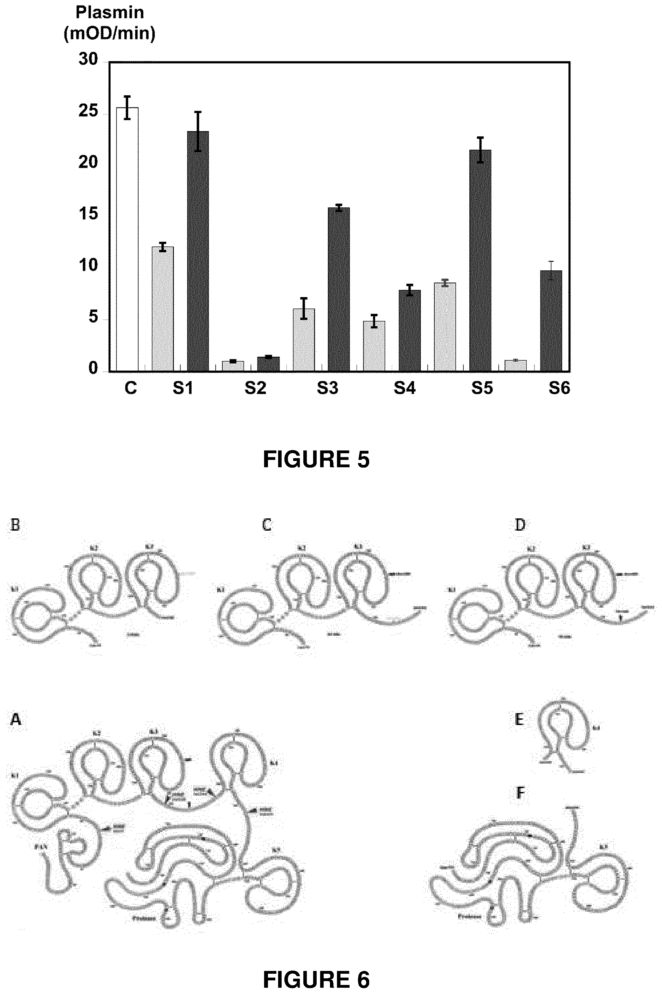

- **Figure 5.** Represents the Fibrinolytic activity of plasma isolated from septic shock. The fibrinolytic activity of plasma was tested by incubating the corresponding euglobulin fraction with fibrin surfaces to which tPA (50 iu/ml) was previously bound. The euglobulin fraction contains plasminogen and plasminogen fragments bearing K1, K4 and K5 as identified by mass spectrometry (see text and Supplemental file). The amount of plasmin formed was quantified by measuring the change in absorbance at A405nm using a chromogenic substrate selective for plasmin as indicated in figure 3. C (open bar): pool of control plasmas ((n=18, plasminogen concentration: 1.5 μM). S1 to S6: samples from septic shock patients [Plasminogen] (μM): S1 (0.546), S2 (0.863), S3 (0.415), S4 (0,491), S5 (0.372), S6 (0.504). Grey bars: activity of S1 to S6 plasma samples at their native plasminogen concentration. Closed bars: activity of S1 to S6 plasma samples upon normalization of the plasminogen concentration to 1.5 μM.

- **Figure 6** represents NETs DNA-HNE complexes digest plasminogen into fragments of known structure identified by proteomic analysis. Plasminogen and its fragments were identified in purified samples and plasma protein fractions by mass spectrometry as indicated above. Schematic representation of plasminogen and its main fragments, modified from the plasminogen structure sequence (http://www.chem.cmu.edu/groups/Llinas/res/structure/kringle-big.html). Figure 6A represents Full-length plasminogen consists of an N-terminal region, 5 kringle (K) domains and the serine protease (SP) region. Arrows indicate the main HNE cleavage sites in plasminogen.(68), Figure 6 B. 27kDa K1-K3 Leu74-Val338, Figure 6 C. 34kDa K1-K3 Leu74-Val354, Figure 6 D. 39kDa K1-K3 Leu74-Val354, Figure 6E.14kDa K4 Val355-Val443 and Figure 6 F. 38kDa K5-SP Ala444 -Asp791. Differences in molecular mass for $K_{1+2+3}$ are related to the site of cleavage and glycosylation.

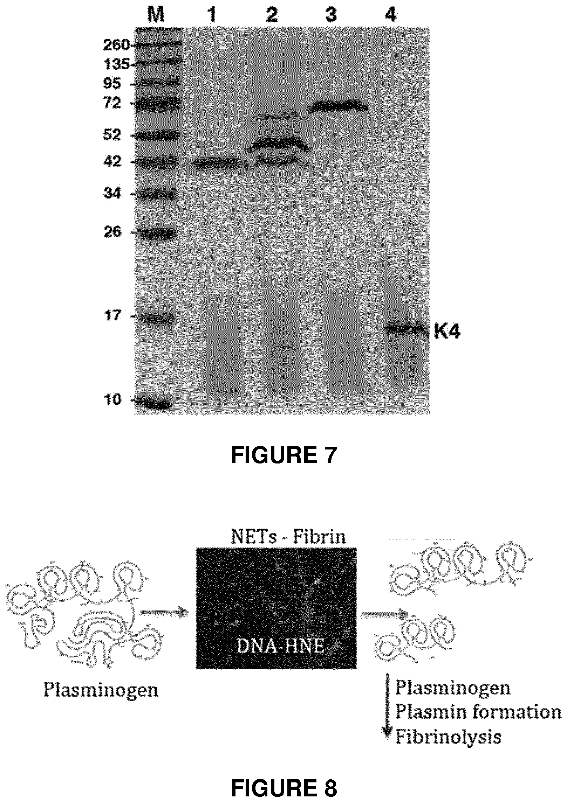

- **Figure 7** represents a photography of Western Blot of Human Neutrophil Elastase-derived fragments of Glu-plasminogen. Fragments were prepared by incubating for 30 min at 37 °C, 10 μM of the purified protein with 250 nM of purified HNE sufficient to fully transform plasminogen into fragments. Corresponding fragments were isolated by affinity chromatography on Lysine-Sepharose and were identified by proteomic analysis using mass spectrometry. M. Molecular markers of reference. 1. K5-SP. 2. First band K1+2+3, second and third bands K1+2+3. 3. K1+2+3+4, 4.

K4

- **Figure 8** is a graph representing plasminogen reduced to fragments by active HNE-DNA complexes of NETs. Plasminogen is reduced to fragments by active HNE-DNA complexes of NETs. HNE-DNA complexes are at the center of a mechanism resulting in severe fibrinolytic failure via the consumption of plasminogen by proteolysis leading to the production of antifibrinolytic plasminogen fragments. This fibrinolytic insufficiency is in part responsible for impaired dissolution of microthrombi thereby fostering their stabilization in the microcirculation and contributing to organ failure during septic shock.

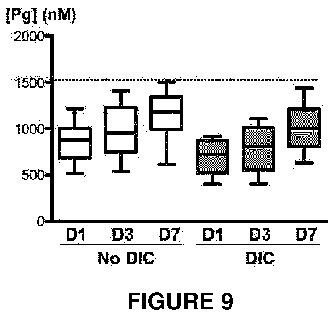

- **Figure 9** represents functional plasminogen concentration in septic shock patients without (No DIC) or with disseminated intravascular coagulation (DIC). Plasminogen was assessed by a functional assay method that measures plasmin formation from available native plasminogen. D1, D3, D7: days after admission to the Intensive Care Unit. The dashed line represents the mean plasminogen concentration of healthy subjects (n=31)

**EXEMPLES**

**Example 1** : **Determination of the concentration of Plasminogen and detection of DIC.**

Activation of platelets and neutrophils in septic shock results in the formation of microvascular clots containing an intricate scaffold of fibrin with neutrophil extracellular traps (NETs). NETs contain multiple components that might impact endogenous fibrinolysis resulting in failure to lyse clots in the microcirculation and residual systemic microthrombosis. Fibrin-NETs matrices were prepared by seeding and activating neutrophils onto a fibrin surface, and monitored plasminogen activation or degradation was constructed. We demonstrate that the elastase activity of HNE-DNA complexes is protected from inhibition by plasma antiproteases and sustain its ability to degrade plasminogen. Using mass spectrometry proteomic analysis, plasminogen fragments composed of kringle (K) domains ($K_{1+2+3}$, $k_{1+2+3+4}$) and the serine protease (SP) region (K5-SP) were identified. The inventors further demonstrate that septic shock patients with disseminated intravascular coagulation have circulating HNE-DNA complexes, HNE-derived plasminogen fragments, a low plasminogen concentration, and a reduced capacity to generate plasmin onto fibrin. The inventors demonstrate that NETs bearing active HNE-DNA complexes reduce plasminogen into fragments thus impairing fibrinolysis by decreasing the local plasminogen concentration, plasminogen binding to fibrin and localized plasmin formation. Thus the reservoir of human neutrophil elastase (HNE) on NETs directly interfere with the fibrinolytic mechanism via a plasminogen proteolytic pathway.

**MATERIALS AND METHODS**

**Abbreviations:**

ABTS, 2,2'-azino-bis(3-ethylbenzothiazoline-6-sulphonic acid)
AEBSF, aminoethyl-benzene-sulfonyl fluoride
$\alpha$1-PI, $\alpha$1-proteinase inhibitor
DIC, disseminated intravascular coagulation
HBSS, Hank's balanced salt solution (HBSS)
HNE, human neutrophil elastase
K, kringle domain of plasminogen
NETs, neutrophil extracellular traps
MeO-Suc-AAPV-CMK, N-(Methoxysuccinyl)-L-alanyl-L-alanyl-L--prolyl-L-valine chloromethylketone
MeO-Suc-AAPV-pNA, N-(Methoxysuccinyl)-L-alanyl-L-alanyl-L--prolyl-L-valine p-nitroanilide
MM-H-Pro-Arg-pNA, methyl(malonyl)hydroxyprolylarginine -p-nitroanilide, CBS0065
NETs, neutrophil extracellular traps
PMA, phorbol 12-myristate 13-acetate
PMN, polymorphonuclear leukocyte
PMSF, phenylmethylsulphonyl fluoride
tPA, tissue plasminogen activator
uPA, urokinase plasminogen activator
uPAR, urokinase plasminogen activator receptor

**Plasma from septic shock patients and healthy subjects**

Seven patients (mean age 74.5±10 years) referred for septic shock with multiple organ failure (mean Sequential Organ Failure Assessment SOFA score 13±2) (30) and presenting disseminated intravascular coagulation (DIC) diagnosed during the first 24 h after admission according to the Japanese Association for Acute Medicine JAAM 2016 score (31) were enrolled, as well as ten healthy individuals (Trial Registration: Clinicaltrial.gov identifier NCT #02391792). The Strasbourg University Hospital Ethics Committee approved this study. Informed consent was obtained from the patient or relatives at admission and confirmed by the patient as soon as possible. Platelet-free plasma was prepared from blood collected in 0.13 M sodium citrate (Vacutainer™, Becton Dickinson) by a double centrifugation step (2500 g for 15 minutes), aliquoted and immediately frozen at -80 °C. Regular haemostasis studies, and detection of HNE-$\alpha$1PI and plasmin-$\alpha$2-antiplasmin complexes were assessed in human plasmas.

**Human proteins and antibodies**

Human Glu-plasminogen was purified as described and was over 99% pure as assessed by SDS/PAGE and by amino-terminal sequence analysis (3, 32). Plasminogen elastase-derived fragments of plasminogen were prepared by incubating for 30 min at 37 °C, 10 $\mu$M of the purified protein with 250 nM of purified HNE. The reaction was stopped by addition of 5 mM PMSF. Fragments were then isolated by affinity chromatography on Lysine-Sepharose. Fibrinogen was purified from fresh-frozen human plasma and characterized as described. (33) Human tPA (>95% single-chain) was obtained from Biopool (Uppsala, Sweden). HNE and the elastase inhibitor $\alpha$1PI were from Calbiochem (Merck KGaA, Darmstadt, Germany). A sheep polyclonal antibody directed against plasminogen and a monoclonal IgG1 antibody directed against plasminogen kringle 1 (CPL15) were produced and characterized previously.(34, 35) Horseradish peroxidase (HRP)-conjugated CPL15 mAb was obtained using a peroxidase labelling kit according to the manufacturer's instructions (Roche, Mannheim, Germany). The polyclonal rabbit anti-sheep (HRP)-conjugated IgG was from DakoCytomation (Glostrup, Denmark).

**Neutrophil Isolation and generation of neutrophil extracellular traps**

Venous blood from healthy volunteers was collected on Acid-Citrate-Dextrose by the Blood Donor Center (Etablissement Frangais du Sang). Neutrophils were isolated from the anticoagulated blood as described. (36) Briefly, leukocytes were separated from red cells on a separating medium containing 9% Dextran T-500 in Radioselectan. After red cell sedimentation, the leukocyte suspension was centrifuged on Pancoll 1077. The cell pellet was washed with phosphate-buffered saline and contaminating erythrocytes were removed by hypotonic lysis. Polymorphonuclear neutrophils (PMN) were then resuspended in RPMI-1640 or Hank's balanced salt solution (HBSS) (see below) and used immediately after isolation. Flow cytometry showed the absence of CD14+, CD3+, CD19+ cells, confirming the recovery of highly purified PMN (CD16+) that were identified by nuclear morphology on May-Grünwald-Giemsa staining (not shown). Routinely, the purity of neutrophil preparations and cell viability (Trypan blue) were ≥ 98%. Isolated neutrophils were re-suspended in RPMI-1640 medium or HBSS at a concentration allowing distribution of 200 000 cells per well. Experiments were performed on 96-well flat-bottomed plates containing or not a well-defined fibrin surface prepared and characterized as described below.

Since PMA and bacteria use related pathway (protein kinase C activation and generation of reactive oxygen species for induction of proteolytically active NETs), we used the well standardized technique of PMA stimulation.(14) After 1 hour, plated neutrophils were treated with 50 nM PMA in a humidified 5% CO2 atmosphere at 37 °C, during 4 hours. NETs were identified by (1) DNA staining with a 10 $\mu$g/ml solution of Hoechst 33342 and (2) measuring the activity of DNA-bound elastase.

Stained nuclei of non-stimulated neutrophils and PMA-induced extracellular traps were detected in optic fields of three wells for each condition using the X20 objective of a Zeiss AxioObserver D1 fluorescence microscope equipped with a CCD Imaging camera using the Histolab software from Microvision Instruments (Evry, France).

To detect elastase bound to NETs, the substrate MeO-Suc-AAPV-pNA (100 $\mu$l per well, 1.5 mM final concentration in HBSS) was incubated with NETs after two gentle washes with HBSS. DNA-bound HNE activity was detected in a multi-well plate counter at 37 °C by measuring the release of p-nitroaniline (pNA) at A405nm. The elastase inhibitors MeO-Suc-AAPV-CMK (100 $\mu$M final) or $\alpha$1-PI (10 $\mu$M final) and AEBSF (1 mM) were used to authenticate preservation of DNA-bound elastase activity.

**Preparation and characterization of fibrin matrices**

Fibrin matrices were prepared as described previously (WO/1985/004425). (34, 37, 38) Briefly, a monolayer of fibrinogen was immobilised onto poly(glutaraldehyde)-activated flat-bottomed 96-well plates. The immobilised fibrinogen

($410 \pm 4$ fmol/cm$^2$) was transformed into fibrin using a 10 N.I.H. u/ml solution of thrombin containing 2 mM CaCl$_2$. The release of fibrinopeptide A was followed using the monoclonal antibody Y18.(39) Fibrin thus obtained specifically interacts with the finger domain of tPA whereas plasminogen binds to newly exposed carboxy-terminal lysine residues unveiled during on-going fibrin degradation.(3, 33, 37) Fibrin degradation was followed with mAb DD3B6 specific for D-dimer structures unveiled by plasmin on the immobilised fibrin.(40) Advantageously fibrin-NETs matrices could be alternatively prepared using a variety of supports, for example beads or slides.

**Reagents**

The chromogenic substrate selective for plasmin (methylmalonyl)hydroxyprolylarginine -p-nitroanilide (CBS0065) was kindly provided by G. Contant (Diagnostica Stago, Asnières, France). Phorbol 12-myristate 13-acetate (PMA), the serine protease inhibitors aminoethyl-benzene-sulfonyl fluoride (AEBSF) and phenylmethylsulphonyl fluoride (PMSF), the elastase-specific substrate N-methoxysuccinyl-Ala-Ala-Pro-Val-p-nitroanilide (MeO-Suc-AAPV-$p$NA) and the elastase-specific inhibitor N-methoxysuccinyl-L-alanyl-L-alanyl-L-prolyl-valine-chloromethyl ketone (MeO-Suc-AAPV-$CMK$) were from Sigma-Aldrich (L'Isle d'Abeau-Chenes, France). Hank's balanced salt solution (HBSS) was from Gibco. Dextran T-500 from Serva. Radioselectan was from Schering, France. Pancoll 1.077 mg/ml was from PAN BIOTECH GmbH (distributed by Dutscher, Brumath, France). Hoechst 33342, a minor groove binder of dsDNA was from BioProbes (distributed by ThermoFisher, France).

**Detection of HNE-$\alpha_1$-PI and plasmin-$\alpha_2$-antiplasmin complexes**

Human neutrophil elastase (HNE) was detected as a complex with the $\alpha_1$-proteinase inhibitor ($\alpha_1$-PI) using the Human PMN Elastase Platinum ELISA kit from Affymetrix (Bender MedSystems GmbH, Vienna, Austria). Plasmin-$\alpha_2$-antiplasmin complexes were detected by ELISA (Technozym, Technoclone GmnH, Vienna, Austria).

**Identification of plasminogen and its fragments by Mass Spectrometry Database search and interpretation of MS datasets**

Samples used were either purified proteins (human plasminogen, plasmin and HNE-derived plasminogen fragments) or plasma protein fractions of interest (euglobulins and lysine-affinity fractions) depleted in highly abundant proteins. The euglobulin fraction from 100 $\mu$l of plasma (patients or control) was precipitated at low ionic strength and pH 5.8 in the presence of protease inhibitors and resuspended in 2.5% SDS, 62.5 mMTris-HCl pH 6.8 sample buffer; this fraction is depleted in albumin and $\alpha$-globulins but contains all $\beta$- and $\gamma$-globulins including plasminogen and its fragments. Proteins bound to lysine-Sepharose from 3 ml of plasma (pool of plasma from 6 patients) were eluted with 30 mM $\epsilon$-aminocaproic acid as previously described.(66) The lysine-affinity fraction specifically contains proteins bearing a lysine-binding site.

Proteins in samples were separated on either 8% or 15% SDS-PAGE gels. Bands were manually excised from gels and were cut into cubes. Then, in-gel digestion was carried out with trypsin, according to a published procedure with minor adjustments (67): sample were destained twice with a mixture of 100 mM ammonium bicarbonate (ABC) and 50% (vol/vol) acetonitrile (ACN) for 30 min at room temperature and then dehydrated using 100% ACN for 20 min, before being reduced with 25 mM ABC containing 10 mM DTT for 1 h at 57 °C and alkylated with 55 mM iodoacetamide in 25 mM ABC for 30 min in the dark at room temperature. Gel pieces were washed twice with 25 mM ABC and dehydrated (twice, 20 min) with 100% ACN. Gel cubes were incubated with sequencing grade-modified trypsin (Promega, USA; 12.5 ng/$\mu$l in 40 mM ABC with 10% ACN, pH 8.0) overnight at 37 °C. After digestion, peptides were extracted twice from gel pieces with a mixture of 50% ACN - 5% formic acid (FA) and then with 100% ACN. Extracts were dried using a vacuum centrifuge concentrator plus (Eppendorf).

Mass spectrometry analyses were performed using an Ultimate 3000 Rapid Separation Liquid Chromatographic (RSLC) system (Thermo Fisher Scientific) online with a Q Exactive hybrid quadrupole Orbitrap mass spectrometer (ThermoFisher Scientific). Briefly, peptides were solubilized in 3.5 $\mu$L of 10% ACN - 0.1% TriFluoroAcetic acid (TFA). Then, peptides were loaded and washed on a C18 reverse-phase precolumn (3 $\mu$m particle size, 100 Å pore size, 75 $\mu$m i.d., 2 cm length). The loading buffer contained 98% H$_2$O, 2% ACN and 0.1% TFA. Peptides were then separated on a C18 reverse-phase resin (2 $\mu$m particle size, 100 Å pore size, 75 $\mu$m i.d., 25 cm length) with a 30 min gradient from 99% A (0.1% FA and 100% H$_2$O) to 40 % B (80% ACN, 0.085% FA and 20% H$_2$O).

The mass spectrometer acquired data throughout the elution process and operated in a data-dependent scheme with full MS scans acquired with the Orbitrap, followed by up to 10 MS/MS HCD spectra in the Orbitrap. Mass spectrometer settings were: full MS (AGC: $3 \times 10e6$, resolution: $7 \times 10e4$, m/z range 400-2000, maximum ion injection time: 100 ms) and MS/MS (AGC: $1 \times 10e5$, maximum injection time: 100 ms, isolation window: 4m/z, dynamic exclusion time setting: 15 s). The fragmentation was permitted for precursors with a charge state of +2, 3 and 4. For the spectral processing, the software used to generate .mgf files was Proteome discoverer 1.4 (ThermoFisher Scientific). Database searches were carried out using Mascot version 2.5.1 (Matrix Science, London, UK) on 'human' proteins (20,273 sequences) from the SwissProt databank containing 550,552 sequences (196,472,675 residues) (February 2016). The search parameters were as follows: the enzyme specificity as semiTrypsin, carbamidomethylation as a fixed modification for cysteins and oxidation as a variable modification for methionines. Up to 1 missed cleavage was tolerated, and mass accuracy tolerance levels of 4 ppm for precursors and 20 mmu for fragments were used for all tryptic mass searches. Positive identification was based on a Mascot score above the significance level (i.e. 5%). The reported proteins were always those with the highest number of peptide matches.

**Study of plasminogen activation/fragmentation on fibrin-NETs matrices**

Fibrin-NETs matrices were achieved by activation with PMA of neutrophils seeded onto fibrin surfaces prepared as described above. The neutrophils were seeded onto the fibrin matrix at 200 000 cells per well and were stimulated with PMA after 1 h plating as indicated above. After 4 h of stimulation a fibrin-NETs matrix was obtained as visualized by Hoechst staining and elastase activity detection (Figure 1). Following the formation of NETs, the supernatant solution was removed, the wells were carefully washed once with HBSS, and the following experiments were performed:

**1.** Plasminogen activation onto the fibrin-NET matrices. After washing with HBSS, tPA at 50 i.u./ml was incubated with the surfaces for 1 h at 37°C to allow binding of tPA to fibrin. Unbound tPA was carefully discarded and a solution of plasminogen at varying concentrations was added to trigger plasmin formation by tPA bound to the fibrin-NETs lattice. In parallel experiments the effect of elastase on plasminogen fragmentation was blocked with the elastase inhibitor MeO-Suc-AAPV-CMK. Kinetics of plasminogen transformation into plasmin was followed during 1 h by measuring the release of pNA at A405nm in a microplate counter. Initial velocities of the reaction were calculated using an ad hoc computer program.

**2.** Plasminogen fragmentation by NETs-bound Elastase. Following the formation of NETs and a wash with HBSS, plasminogen at 1 $\mu$M was incubated with NETs at 37 °C for 15 min to 4 hours. When indicated the NETs were pre-treated with the elastase inhibitor MeO-Suc-AAPV-CMK or $\alpha$1-PI before addition of plasminogen. Post incubation, the plate was centrifuged at 3000 g for 5 min and the supernatants from each well were collected and stored at -20 °C for further analysis. Material remaining at the bottom onto the fibrin surface was recovered by scraping using 100 $\mu$l of sample buffer (60 mM Tris-HCl pH 6.8, 2% SDS) or 100$\mu$l of HBSS, and used for Western blotting or detection of HNE activity, respectively. HNE activity in samples collected in HBSS was measured with the chromogenic substrate MeO-Suc-AAPV-pNA (100 $\mu$l per well, 1.5 mM final concentration in HBSS) as indicated above. Plasminogen proteolysis was investigated in samples collected in Tris-SDS by SDS-10% PAGE followed by Western blotting using a mAb directed against plasminogen K1 (CPL15-HRP, 100 ng/ml)(35) or a polyclonal sheep IgG (125 $\mu$g/ml) followed by a secondary anti-sheep IgG-HRP (1:20 000). Signal was detected using the ECL kit from Amersham (Arlington Heights, U.K.) and the Curix-60-AGFA for development.

**Detection of plasminogen, plasminogen fragments and plasmin formation in plasma from septic shock patients**

Plasminogen was quantified with a functional activity assay as described with modifications.(41) Briefly, plasmas were diluted 1:50 in assay buffer (Phosphate 80 mM, NaCl 50 mM, BSA 2 mg/ml), supplemented with 75 i.u./ml urokinase, 1 mM tranexamic acid and 0.75 mM of the plasmin-selective chromogenic substrate in a final volume of 50 $\mu$l. Kinetics of the reaction was followed by measuring the release of pNA at A405nm in a microplate photometer in kinetics mode. Results were given in nM concentration by reference to a standard curve prepared with plasminogen-depleted plasma supplemented with known concentrations of purified plasminogen (0.2 to 1 $\mu$M). Plasminogen fragments present in plasma were initially detected by Western blot as indicated above. Further identification was obtained by mass spectrometric (see above). Plasmin formation on fibrin matrices was measured using the euglobulin fraction from healthy control and patient plasmas as source of plasminogen as indicated above. Plasminogen activation onto the fibrin-NET matrices. Kinetics of plasminogen transformation into plasmin was followed during 1 h by measuring the release of pNA at A405nm and the initial velocity of the reactions were calculated as indicated above.

**Neutrophil extracellular traps detection in human plasmas**

Plasma HNE-DNA complexes of NETs were identified using an ELISA capture assay as described (19) with the following modifications. Since HNE is the enzyme involved in plasminogen fragmentation and is also the most abundant granular component of NETs, we choose to capture HNE-DNA complexes using an anti-HNE antibody. The antibody (10 $\mu$g/ml) was immobilized on poly(glutaraldehyde)-activated U 96-well polyvinyl chloride plates as described above for fibrinogen. A volume of 50 μl of diluted human plasma was added per well in combination with the peroxidase-labeled anti-DNA monoclonal antibody (component No.2 of the commercial cell death detection ELISA kit) according to the manufacturer's instructions (Roche Diagnostic, Mannheim, Germany). After 1 h incubation at 37 °C, the samples were washed three times with 100μl PBS per well and 100μl/well of the peroxidase substrate ABTS at 1 mg/ml were added. The absorbance at 405 nm wavelength was measured at 37 °C in the dark using a multi-well plate photometer as indicated above.

## RESULTS:

### NETs released onto a fibrin matrix form a dense lattice

NETs were identified as extracellular fibrillar networks primarily composed of DNA stained by Hoechst dye. Further identification was achieved by detecting HNE bound to DNA using an activity assay (Figure 1). Non-stimulated neutrophils presented typical intracellular lobulated nuclei easily recognized either before or after 4 h incubation at 37 °C on plates containing or not a fibrin matrix (Figure1A, Neutrophils). Extracellular DNA could not be observed in non-stimulated neutrophils indicating that fibrin alone was not competent to boost the formation and release of NETs. NETs released under similar conditions in the absence of fibrin appeared isolated and did not knit (Figure 1A, NETs) In contrast, extracellular DNA released by stimulated neutrophils seeded on fibrin formed a dense interlace of fibers clearly visible after Hoechst-staining (Figure 1A, NETs-Fibrin), suggesting that DNA spreading on fibrin was favored by the regular distribution of positive charges along the fibrin fibers.(42) Of note that fibrin alone display no autofluorescence (not shown) that may influence the dense appearance of NETs. The presence of HNE coupled to NETs was detected with the elastase-selective chromogenic substrate MeO-Suc-AAPV-pNA (Figure 1B). The proteolytic activity of HNE-DNA complexes was completely blocked by the peptidyl chloromethyl ketone elastase inhibitor MeO-Suc-AAPV-CMK (Figure 1B). In contrast, $\alpha_1$-PI, which efficiently inhibits soluble HNE in supernatants or added to plasma (Figure 1C), produced only around 20% decrease in the activity of DNA-associated HNE (Figure 1B). Aprotinin, an unrelated inhibitor, was without effect on the activity of HNE when added to NETs or cell supernatants at a similar concentration than α1-PI (40 μM) (Figure 1B). These data indicate that HNE-DNA lattices spread onto the fibrin matrix is fully active.

### NETs' HNE•DNA complexes cleave plasminogen into well-defined fragments

It is well-known that HNE in solution (purified or released by neutrophils) produces plasminogen fragments of known structure (4, 44). However, it remains unknown whether HNE bound to NETs is able to cleave plasminogen into fragments. To investigate this hypothesis, we incubated plasminogen with lattices of NETs spread on the fibrin matrix. Figure 2A, clearly shows that plasminogen fragments were already present after 30 min and 1 h of incubation with NETs. Three main bands with a relative molecular mass of 92 kDa, 47 kDa and 32 kDa were observed as well as an additional small band of around 50 kDa on top of the 47 kDa band. Longer incubation times did not modify this pattern (Fig 2B). Proteomic analysis identified the 92 kDa band as human plasminogen, the 47 and 50 kDa bands as fragment K1+2+3, and the 32 kDa band as K5-SP (figure 6). K4 was clearly identified by proteomic analysis in NETs-HNE-treated plasminogen (figure 6) and in purified samples of HNE-treated plasminogen (figure 7). HNE-DNA complexes specifically cleave plasminogen by pre-incubating NETs with selective proteinase inhibitors was verified. Pre-incubation with MeO-Suc-AAPV-CMK, an elastase-specific inhibitor, completely inhibited HNE and abolished the conversion of plasminogen into fragments confirming that HNE was active on NETs (Figure 2C). In contrast, aprotinin, a plasmin and kallikrein inhibitor, was unable to modify the activity of elastase or the appearance of plasminogen fragments (Figure 1B). Because, α1-PI the specific inhibitor of elastase had only a minor reducing effect on the activity of HNE-DNA complexes (Figure 1B), demonstrating that the observed plasminogen fragments are generated by the proteolytic activity of HNE-DNA complexes.

### NETs HNE-derived plasminogen fragments interfere with fibrinolysis

Having demonstrated that HNE•DNA complexes of NETs cleave plasminogen into fragments K1+2+3, K4 and K5-SP, the critical question of their role in fibrinolysis was sought to be answer. For that purpose, neutrophils seeded onto a fibrin matrix were prompted to release NETs with PMA as above. Conversion of plasminogen into plasmin by tPA bound to the fibrin-NET lattice was then assessed using a plasmin-selective chromogenic substrate. The time-and concentration-dependent conversion of plasminogen into plasmin at the fibrin-NETs lattice was reduced as compared to fibrin alone (Figure 3A-B). Plasmin formation was restored to control values when MeO-Suc-AAPV-CMK, an elastase-selective inhibitor, was pre-incubated with the fibrin-NET lattice, indicating that HNE proteolytic activity prevents plasminogen conversion into plasmin. The amount of plasmin formed and remaining bound to the fibrin-NETs lattices was decreased by around 50% as compared to similar conditions in the presence of MeO-Suc-AAPV-CMK (Figure 3C). This decrease in the amount of plasmin formed onto fibrin was related to plasminogen fragmentation by HNE•DNA complexes as indicated by Western blot identification of the molecular species eluted from the fibrin surfaces (Figure 3D). Altogether, data in figure 3 show that plasminogen fragments were identified in fibrin matrices containing HNE•DNA complexes and low fibrinolytic activity. In contrast, plasminogen fragments were absent and the fibrinolytic activity was similar to control fibrin when fibrin-NETs matrices were pre-treated with MeO-Suc-AAPV-CMK.

**NETs, plasminogen fragments, HNE•$\alpha$1-PI and fibrinolytic activity in septic shock plasma.**

Results of the parameters tested are shown in Table 1 below.

Table 1. Fibrinolytic parameters and NETs in septic shock and healthy control plasmas

| Patient's samples (S) | HNE•$\alpha_1$-PI ng/ml | plasmin-$\alpha_2$-antiplasmin ng/ml | NETs HNE-DNA A405nm | Plaminogen $\mu$M |
|---|---|---|---|---|
| S1 | 223 | 1245 | 0,211 | 0,546 |
| S2 | 230 | 366 | 0,222 | 0,863 |
| S3 | 320 | 1654 | 0,302 | 0,415 |
| S4 | 765 | 4814 | 0,284 | 0,491 |
| S5 | 212 | 1207 | 0,284 | 0,372 |
| S6 | 177 | 159 | 0,325 | 0,504 |
| S7 | 935 | 720 | 0,302 | 0,689 |
| Mean$\pm$SD | 437$\pm$274 | 1534$\pm$1517 | 0,276$\pm$0,043 | 0,554$\pm$0,170 |
| Range | 177-935 | 159- 4814 | 0,211- 0,325 | 0,372- 0,863 |
| Median | 320 | 1207 | 0,284 | 0,504 |
| Healthy Controls | n=31 | n=24 | N=5 | N=31 |
| Mean$\pm$SD | 43$\pm$19 | 89$\pm$76 | 0,086,014 | 1,69$\pm$0,33 |
| Range | 7-85 | 10-240 | 0,065-0,100 | 1,23-2,28 |
| Median | 45 | 59 | 0,086 | 1,65 |
| Pool 91 | 45 | 96 | ND | 1,72$\pm$0,13 |
| Pool 91: pool constituted with plasma separated from blood obtained from 18 healthy controls. ND: not done. | | | | |

NETS were detected by measuring HNE•DNA complexes in samples from patients with septic shock and disseminated intravascular coagulation (patients 0.276$\pm$0.043; healthy controls: 0.086$\pm$0.014, A405nm). The concentration of plasminogen as measured with a functional assay was 520$\pm$180 nM (healthy controls n=31: 1.69$\pm$0.33 $\mu$M). Circulating HNE•$\alpha$1-PI complexes were increased 8-fold in patients as compared to healthy controls, 437$\pm$274 versus 43$\pm$19 ng/ml, respectively. Patients had elevated D-dimers (12$\pm$8 mg/l) and plasmin-$\alpha$2-antiplasmin (range 4814-159 ng/ml, median 1207 ng/ml), without signs of hepatic dysfunction, although moderate cytolysis. The presence of HNE-derived plasminogen fragments was evidenced by SDS-PAGE and Western blotting. Figure 4 shows the pattern of plasminogen fragments detected in 7 selected patients as compared to healthy controls. Besides plasminogen, three main plasminogen fragments were identified by mass spectrometric analysis of amino-acid sequence of protein bands from samples depleted of highly abundant proteins (euglobulin fractions or lysine-affinity fractions): plasminogen fragments containing K1+2+3, K1+2+3+4 and plasminogen fragments composed of K5-SP (figure 6). K4 was not detected in plasma samples of septic shock patients despite the use of acrylamide gels of small pore size or shorter migration time. In contrast K4 was clearly identified in purified plasminogen treated by NETs or HNE alone (figures 6 -7).

To investigate the effect of NETs and plasminogen fragments on fibrinolysis, the euglobulin fraction of septic shock plasma samples were incubated with tPA bound to fibrin surfaces and the amount of plasmin formed was quantified with a chromogenic substrate. The generation of plasmin was decreased (>50%) when comparing different septic shock plasma to a pool of normal plasma (Figure 5). To compensate for the rather low plasminogen concentration of these plasmas, purified plasminogen was added to a concentration similar to that of the normal plasma pool. As a result, the amount of plasmin formed was increased with a trend to normality in only three (S1, S3, S5) of the plasma supplemented with plasminogen. An insufficient or scant response was observed in the remaining plasma samples S2, S4, S6), suggesting a competitive effect towards added plasminogen.

## DISCUSSION

Netosis, a major antimicrobial mechanism, has alternative roles in both infectious and non-infectious diseases. In septic shock, for instance, activated neutrophils emit extracellular traps that both ensnare pathogens and activate coagulation leading to fibrin formation. The microvascular clots that ensue, a mechanism referred to as immunothrombosis, favor pathogens circumscription and killing by NETs enzymes. (20, 45, 46) Several actors of this interconnected mechanism between coagulation and innate immunity may also nurture uncontrolled thrombosis and thereby hinder the initial benefit of the response.(47) As a consequence, NETs may substantially contribute to septic shock-associated intravascular coagulation. Actually, we detected circulating NETs in septic shock patients with intravascular coagulation, (48) a finding that was confirmed in recent studies.(49-52). The example is focus on the role of NETs on the other side of the hemostatic equilibrium: the fibrinolytic response.

Using a fluorescent DNA binder, the inventors notice that NETs formed in vitro onto a fibrin surface show a denser and more reticulated appearance than NETs released in the absence of fibrin. The positive charges regularly distributed along the fibrin fibers interact with negatively charged DNA yielding a more dense appearance of DNA fibers in the entangled fibrin-NETs matrix. These findings agree with data previously reported indicating that cell-free DNA incorporated into a clot modifies the structure of fibrin rendering it resistant to plasmin-mediated degradation.(26, 28) The entangled assembly of DNA and fibrin within the thrombus may be implicated in NETs-mediated resistance to tPA-induced fibrinolysis in vitro (21, 29) and thrombolysis in patients with acute ischemic stroke.(54) Such fibrinolytic resistance may contribute to persisting thrombotic occlusion due to sustained platelet activation by histones (21) and inactivation of tissue factor pathway inhibitor (TFPI) by HNE.(45) Actually, the inventors demonstrate that NETs entangled with fibrin contain active HNE in complex with DNA that is insensitive to inhibition by $\alpha$1-PI. Similarly, NETs-associated murine NE was found to be proteolytically active within the liver vasculature.(55) Contrastingly, elastase bound to the plasma membrane of neutrophils is easily released to form soluble irreversible complexes with circulating $\alpha$1-PI.(7) The protective function of DNA on HNE activity is in agreement with previous findings indicating that leukocyte granular serine proteases bind to DNA with nanomolar affinity and endow HNE with resistance to plasma inhibitors thus ensuring localized degradation of protein substrates.(9, 11, 12, 17).

The example clearly demonstrates that in the presence of NETs containing active HNE-DNA complexes plasminogen is reduced to fragments of well-defined size: K1+2+3, K1+2+3-4, K4 and K5-SP that have lost their capacity to generate plasmin by fibrin-bound tPA. These plasminogen fragments were detected when plasminogen was added to NETs released by activated neutrophils in-vitro. The nature of the fragments was identified with certainty by mass spectrometric analysis of amino-acid sequences. Since plasminogen fragments bearing K1 have been previously shown to be strong inhibitors of plasminogen binding to fibrin, plasmin generation and fibrinolysis,(56-58) the inventors investigate the role of NETs on these mechanisms. The experimental fibrin-NETs lattice used by the inventors is an outstanding set-up suitable to detect both the NETs-HNE-derived plasminogen fragments and the competitive effect of K1-containing fragments on plasminogen binding and activation. The inventors demonstrate that when plasminogen was activated by fibrin-bound tPA on the NETs-fibrin lattice, a decrease in the amount of plasmin generated by tPA occurred concomitantly with the appearance of such fragments. These data indicate that these plasminogen fragments compete with plasminogen for binding to fibrin decreasing thereby its capacity to generate plasmin.

At variance with the antifibrinolytic effect described here, there are other protein components of hemostasis that could be a substrate for HNE-some of which may have direct or indirect effects on thrombus formation and lysis. In particular, HNE may exert either a procoagulant activity by inactivating TFPI(45) or a profibrinolytic activity by direct cleavage of fibrin,(59) by degrading $\alpha$2-antiplasmin or PAI-1 (60, 61) or by producing mini-plasminogen (K5-SP).(5) Although mini-plasminogen (K5-SP) may be activated by urokinase in solution, it does not bind to fibrin and is therefore not activated by tPA at the fibrin surface; as a consequence, the impact of K5-SP on tPA-fibrin-dependent clot lysis is negligible.(5, 62).

Taken all together, the results clearly demonstrate that HNE-DNA complexes exert an antifibrinolytic effect by generating plasminogen fragments and decreasing the plasminogen concentration.

The indicators of NET-induced fibrinolytic failure in vitro in plasma samples from septic shock patients with DIC and circulating NETs were investigated. The inventors clearly demonstrate that this group of patients have a low plasminogen concentration, circulating plasminogen fragments and a failure to generate plasmin on fibrin (less than 50% when compared to a pool of normal plasmas) which clearly contribute and/or be responsible for the observed intravascular coagulation and multiple organ dysfunction. Protein bands detected by Western blot and identified by mass spectrometric amino-acid analysis in plasma fraction of the selected patients corresponded to intact plasminogen and plasminogen fragments: K1+2+3, K1+2+3+4 and K5-SP. These fragments were identical to those derived from plasminogen cleaved in vitro by purified HNE.(44).

Since $\alpha$1-PI circulates at $\mu$M concentration and inhibits all free HNE with an extremely high association constant (Ka = 6.5 × 107 M-1 s-1),(6) elastase released by neutrophils was readily found as HNE-α1PI complexes in plasma from septic shock patients. HNE-α1PI complexes are therefore a marker of neutrophil activation in sepsis. The inventors demonstrate that in the absence of active elastase in the plasma of these patients, the plasminogen fragments found in plasma were produced by active HNE-DNA complexes. These data, strongly support a mechanistic pathway where plasminogen fragments found in patients with septic shock were produced locally on the fibrin-NETs matrix of the clot. Nevertheless, besides fibrin, histone H2B bearing carboxyterminal lysines may capture plasminogen in the vicinity of HNE bound to the DNA fibers.(64) Circulating NETs containing active HNE-DNA complexes insensitive to α1-PI could also participate in plasminogen fragmentation.

As demonstrated, the proteolysis of plasminogen by HNE-DNA complexes is in part responsible for the low concentration, less than 1μM, of full-length plasminogen detected in the plasma of septic shock patients. The low concentration of plasminogen in conjunction with the anti-fibrinolytic activity of plasminogen fragments result in insufficient fibrinolysis in septic shock leading to disseminated intravascular coagulation (DIC). These parameters, plasminogen concentration, plasminogen fragments and failure to form plasmin are relevant new prognostic markers to assess the role of NETs in sepsis and septic shock induced DIC and serve as predictors of mortality and organ failure in septic shock patients.

Since the low plasminogen concentration of these plasmas could explain in part the low fibrinolytic activity, the plasmas were supplemented with plasminogen before testing. In spite of reaching a plasminogen concentration similar to that of the normal plasma pool, 3 out of the 6 plasmas tested failed to restore their fibrinolytic activity to normal values. These results demonstrate a competitive effect towards plasminogen most probably related to the presence of circulating HNE-derived plasminogen fragments containing K1.

The results clearly demonstrate that a severe decrease in fibrinolysis induced by HNE-DNA complexes of NETs contribute to the persistence and stabilization of thrombi in the microcirculation during septic shock.

The inventors also demonstrate that septic shock thrombosis in the microcirculation is in relation with the capacity of HNE-DNA complexes to trigger fibrinolytic insufficiency. Identification of this reduction in the fibrinolytic potential in plasma of septic shock patients, as shown in figure 5, have practical implications in the search for a feasible alternative to improve the fibrinolytic response in these patients. For instance, the active enzyme i.e. HNE-DNA complexes, and the substrate, i.e. plasminogen, represent new targets and/or compound useful in the therapy of sepsis-DIC.

The inventors demonstrate that NETs or fragments of NETs contain active elastase responsible for the production of plasminogen fragments with inhibitory activity (competitors of plasminogen binding to fibrin), which act to decrease both the concentration of plasminogen and plasmin formation. Altogether, the low plasminogen and the inhibitory activity of the elastase-derived plasminogen fragments contribute to abrogate the fibrinolytic system, thus favoring microthrombosis and organ failure.

The example clearly demonstrates that the fragmentation of plasminogen by active HNE-DNA complexes and a concomitant decrease in both the concentration of plasminogen, and plasmin formation are required are required to dampen fibrinolysis (Figure 4). It also demonstrates that the low capacity to generate plasmin is related to a decrease in the amount of plasminogen bound to fibrin resulting from both the low plasminogen concentration and a competitive effect due to the elastase-derived plasminogen fragments. The data clearly indicate that HNE-DNA complexes are at the center of a mechanism resulting in severe fibrinolytic failure via the consumption of plasminogen by proteolysis leading to the production of antifibrinolytic plasminogen fragments. This fibrinolytic insufficiency is responsible for impaired dissolution of microthrombi thereby fostering their stabilization in the microcirculation, contributing to DIC and organ failure during septic shock.

This example clearly demonstrates that the level of plasminogen is a biological marker of the fibrinolytic response associated to DIC, in particular during septic shock.

This example also clearly demonstrates that plasminogen restores fibrinolytic activity in patients suffering netosis and septic shock associated DIC and constitute a new therapeutic treatment for these patients by the supplementation.

## Example 2 : Plasminogen, an indicator of fibrinolysis response in sepsis-and septic shock DIC

Plasminogen is the precursor of plasmin, the enzyme required to dissolve blot clots. Plasminogen is transformed into plasmin by the endothelial plasminogen activator tPA. Plasminogen circulates in blood at a concentration ranging from 1.5 to 2 μmol/L (138 μg/ml to 184 μg/ml), which is in excess over the amount required to generate full plasmin activity by tPA on fibrin. However, at concentrations lower than 1 μM plasminogen is a rate-limiting factor for plasmin generation that may lead to insufficient fibrinolysis (Biochem J 1995).

Indeed, previous studies have shown that the rate of thrombus lysis is limited by the amount of available plasminogen that governs its accumulation onto fibrin (Biochemistry 1991, Thromb Haemostas 1991; J Lab Clin Med 1992, Circulation 1995). A continuous plasminogen supply from the circulation is therefore necessary to ensure its accumulation on the thrombus and sustain the potential for its optimal lysis. However, this supply is restricted in pathological conditions where thrombosis in the microcirculation is combined to an important decrease in plasminogen; a compromised blood flow by insufficient thrombus lysis, may thereby worsen the clinical prognosis.

The example 1 above clearly demonstrates that in patients with septic shock presenting disseminated intravascular clot formation (DIC) the functional plasminogen concentration is found below 1 $\mu$M or even lower than 0.5 $\mu$M in severe cases (Figure 9 ).

The decreased plasminogen concentration found in patients with septic shock may result in part from consumption via fibrinolysis secondary to DIC (as indicated by circulating plasmin•a2-antiplasmin complexes and D-dimers plasma concentration). However, the persistence of microthrombi in DIC indicates a failure in the fibrinolytic response.

In septic shock with DIC neutrophils and platelets act in concert to promote coagulation and formation of Neutrophil Extracellular Traps (NETs) in a reciprocal coupling process called immunothrombosis (20, 45). NETs formation is an intermediary mechanism in septic shock (Figure 4).

NETs are DNA fibers bearing elastase, myeloperoxidase and other granular proteins. Unlike free elastase, NET-associated elastase is resistant to the a1-proteinase inhibitor and is therefore able to cleave plasminogen into well-known fragments that have loss its capacity to generate plasmin.

The results clearly demonstrate that the important decrease in the concentration of functional plasminogen detected in septic shock patients with DIC is mainly related to proteolysis by elastase-DNA complexes of NETs. Proteolysis of full-length plasminogen, N-ter region-K1-K2-K3-K4-K5-SP (K: kringle domain, SP: serine-protease region) by elastase gives rise to plasminogen fragments K1+2+3, K1+2+3+4, K4 and K5-SP (also called mini-plasminogen).

The data obtained in a cohort of patients (n=100) show that plasmin generation is importantly decreased in patients with septic shock and low plasminogen as compared to healthy volunteers. The low capacity to form plasmin results in insufficient fibrinolysis that favors the presence of thrombi in the microcirculation (DIC) and the associated multi-organ dysfunction in patients with septic shock. Extensive ischemic tissue damage associated to DIC leads to failure of multiple organs (especially lung, kidney, liver, and brain).

The data on plasmin generation onto the fibrin surface using these plasmas show that mini-plasminogen (K5-SP) beyond being a marker of plasminogen proteolysis by Elastase•DNA is, above all, a source of non-fibrinolytic mini-plasmin, i.e. a contributor of fibrinolytic insufficiency. Plasmas containing mini-plasminogen generate mini-plasmin lacking the ability to bind to fibrin, i.e. easily inhibited by plasma inhibitors and therefore with no specificity for fibrinolysis. As such, K5-SP in plasma interferes with the functional determination of full-length plasminogen and with the assessment of fibrinolysis.

Administration of plasminogen to patients with septic shock having an important decrease of endogenous circulating plasminogen is advantageous to improve effective fibrinolysis, thrombi dissolution and recovery of patient's health. Within this context, plasminogen monitoring (full-length protein and/or its fragments) is a key determinant in assessment of the fibrinolytic response to DIC in septic shock patients and its evolution to organ dysfunction, and in monitoring of plasminogen recovery during on-going replacement therapy with human Glu-plasminogen.

The plasminogen (and/or its fragments) as a key analytic measure in diagnosis and/or follow-up of patients, for example with netosis-associated septic shock, for both the diagnosis of DIC and/or also plasminogen-related fibrinolytic insufficiency and its treatment with plasminogen replacement.

The fibrinolytic and NETs components/parameters are to be assayed in patients with septic shock (n=100) as compared to healthy individuals (n=31)

In addition, the example also clearly supports that the determination of mini-plasminogen (K5-SP) is a direct marker of elastase•DNA activity and is a marker of pathology associated with NETs, for example sepsis, ischemic stroke, coronary thrombosis, cancer, and autoimmune diseases, among others. Assessment of mini-plasminogen by a functional assay may also provide information about its role in fibrinolysis insufficiency.

**References**

1. Longstaff, C., and Kolev, K. (2015) Basic mechanisms and regulation of fibrinolysis. J Thromb Haemost 13 Suppl 1, S98-105

2. Xue, Y., Bodin, C., and Olsson, K. (2012) Crystal structure of the native plasminogen reveals an activation-resistant compact conformation. J Thromb Haemost 10, 1385-1396

3. Fleury, V., and Angles-Cano, E. (1991) Characterization of the binding of plasminogen to fibrin surfaces: the role of carboxy-terminal lysines. Biochemistry-Us 30, 7630-7638

4. Scapini, P., Nesi, L., Morini, M., Tanghetti, E., Belleri, M., Noonan, D., Presta, M., Albini, A., and Cassatella, M. A. (2002) Generation of biologically active angiostatin kringle 1-3 by activated human neutrophils. J Immunol 168, 5798-5804

5. Moroz, L. A. (1981) Mini-plasminogen: a mechanism for leukocyte modulation of plasminogen activation by urokinase. Blood 58, 97-104

6. Beatty, K., Bieth, J., and Travis, J. (1980) Kinetics of association of serine proteinases with native and oxidized alpha-1-proteinase inhibitor and alpha-1-antichymotrypsin. J Biol Chem 255, 3931-3934

7. Korkmaz, B., Attucci, S., Jourdan, M. L., Juliano, L., and Gauthier, F. (2005) Inhibition of neutrophil elastase by alpha1-protease inhibitor at the surface of human polymorphonuclear neutrophils. J Immunol 175, 3329-3338

8. Owen, C. A., Campbell, M. A., Sannes, P. L., Boukedes, S. S., and Campbell, E. J. (1995) Cell surface-bound elastase and cathepsin G on human neutrophils: a novel, non-oxidative mechanism by which neutrophils focus and preserve catalytic activity of serine proteinases. J Cell Biol 131, 775-789

9. Belorgey, D., and Bieth, J. G. (1998) Effect of polynucleotides on the inhibition of neutrophil elastase by mucus proteinase inhibitor and alpha 1-proteinase inhibitor. Biochemistry-Us 37, 16416-16422

10. Belorgey, D., and Bieth, J. G. (1995) DNA binds neutrophil elastase and mucus proteinase inhibitor and impairs their functional activity. FEBS Lett 361, 265-268

11. Duranton, J., Belorgey, D., Carrere, J., Donato, L., Moritz, T., and Bieth, J. G. (2000) Effect of DNase on the activity of neutrophil elastase, cathepsin G and proteinase 3 in the presence of DNA. FEBS Lett 473, 154-156

12. Duranton, J., Boudier, C., Belorgey, D., Mellet, P., and Bieth, J. G. (2000) DNA strongly impairs the inhibition of cathepsin G by alpha(1)-antichymotrypsin and alpha(1)-proteinase inhibitor. J Biol Chem 275, 3787-3792

13. Brinkmann, V., Reichard, U., Goosmann, C., Fauler, B., Uhlemann, Y., Weiss, D. S., Weinrauch, Y., and Zychlinsky, A. (2004) Neutrophil extracellular traps kill bacteria. Science 303, 1532-1535

14. Kenny, E. F., Herzig, A., Kruger, R., Muth, A., Mondal, S., Thompson, P. R., Brinkmann, V., Bernuth, H. V., and Zychlinsky, A. (2017) Diverse stimuli engage different neutrophil extracellular trap pathways. Elife 6

15. Fuchs, T. A., Abed, U., Goosmann, C., Hurwitz, R., Schulze, I., Wahn, V., Weinrauch, Y., Brinkmann, V., and Zychlinsky, A. (2007) Novel cell death program leads to neutrophil extracellular traps. J Cell Biol 176, 231-241

16. Urban, C. F., Ermert, D., Schmid, M., Abu-Abed, U., Goosmann, C., Nacken, W., Brinkmann, V., Jungblut, P. R., and Zychlinsky, A. (2009) Neutrophil extracellular traps contain calprotectin, a cytosolic protein complex involved in host defense against Candida albicans. Plos Pathog 5, e1000639

17. Thomas, M. P., Whangbo, J., McCrossan, G., Deutsch, A. J., Martinod, K., Walch, M., and Lieberman, J. (2014) Leukocyte protease binding to nucleic acids promotes nuclear localization and cleavage of nucleic acid binding proteins. J Immunol 192, 5390-5397

18. Brinkmann, V., and Zychlinsky, A. (2007) Beneficial suicide: why neutrophils die to make NETs. Nat Rev Microbiol 5, 577-582

19. Kessenbrock, K., Krumbholz, M., Schonermarck, U., Back, W., Gross, W. L., Werb, Z., Grone, H. J., Brinkmann, V., and Jenne, D. E. (2009) Netting neutrophils in autoimmune small-vessel vasculitis. Nat Med 15, 623-625

20. Engelmann, B., and Massberg, S. (2013) Thrombosis as an intravascular effector of innate immunity. Nat Rev Immunol 13, 34-45

21. Fuchs, T. A., Brill, A., Duerschmied, D., Schatzberg, D., Monestier, M., Myers, D. D., Jr., Wrobleski, S. K., Wakefield, T. W., Hartwig, J. H., and Wagner, D. D. (2010) Extracellular DNA traps promote thrombosis. Proc Natl Acad Sci U S A 107, 15880-15885

22. Grassle, S., Huck, V., Pappelbaum, K. I., Gorzelanny, C., Aponte-Santamaria, C., Baldauf, C., Grater, F., Schneppenheim, R., Obser, T., and Schneider, S. W. (2014) von Willebrand factor directly interacts with DNA from neutrophil extracellular traps. Arterioscler Thromb Vasc Biol 34, 1382-1389

23. de Boer, O. J., Li, X., Teeling, P., Mackaay, C., Ploegmakers, H. J., van der Loos, C. M., Daemen, M. J., de Winter, R. J., and van der Wal, A. C. (2013) Neutrophils, neutrophil extracellular traps and interleukin-17 associate with the organisation of thrombi in acute myocardial infarction. Thromb Haemost 109, 290-297

24. Riegger, J., Byrne, R. A., Joner, M., Chandraratne, S., Gershlick, A. H., Ten Berg, J. M., Adriaenssens, T., Guagliumi, G., Godschalk, T. C., Neumann, F. J., Trenk, D., Feldman, L. J., Steg, P. G., Desmet, W., Alfonso, F., Goodall, A. H., Wojdyla, R., Dudek, D., Philippi, V., Opinaldo, S., Titova, A., Malik, N., Cotton, J., Jhagroe, D. A., Heestermans, A. A., Sinnaeve, P., Vermeersch, P., Valina, C., Schulz, C., Kastrati, A., Massberg, S., and Prevention of Late Stent Thrombosis by an Interdisciplinary Global European Effort, I. (2016) Histopathological evaluation of thrombus in patients presenting with stent thrombosis. A multicenter European study: a report of the prevention of late stent thrombosis by an interdisciplinary global European effort consortium. Eur Heart J 37, 1538-1549

25. Laridan, E., Denorme, F., Desender, L., Francois, O., Andersson, T., Deckmyn, H., Vanhoorelbeke, K., and De Meyer, S. F. (2017) Neutrophil extracellular traps in ischemic stroke thrombi. Ann Neurol 82, 223-232

26. Longstaff, C., Varju, I., Sotonyi, P., Szabo, L., Krumrey, M., Hoell, A., Bota, A., Varga, Z., Komorowicz, E., and Kolev, K. (2013) Mechanical stability and fibrinolytic resistance of clots containing fibrin, DNA, and histones. J Biol Chem 288, 6946-6956

27. Gould, T. J., Vu, T. T., Stafford, A. R., Dwivedi, D. J., Kim, P. Y., Fox-Robichaud, A. E., Weitz, J. I., and Liaw, P. C. (2015) Cell-Free DNA Modulates Clot Structure and Impairs Fibrinolysis in Sepsis. Arterioscler Thromb Vasc Biol 35, 2544-2553

28. Varju, I., Longstaff, C., Szabo, L., Farkas, A. Z., Varga-Szabo, V. J., Tanka-Salamon, A., Machovich, R., and Kolev, K. (2015) DNA, histones and neutrophil extracellular traps exert anti-fibrinolytic effects in a plasma environment. Thromb Haemost 113, 1289-1298

29. Fuchs, T. A., Brill, A., and Wagner, D. D. (2012) Neutrophil extracellular trap (NET) impact on deep vein thrombosis. Arterioscler Thromb Vasc Biol 32, 1777-1783

30. Singer, M. (2016) The new sepsis consensus definitions (Sepsis-3): the good, the not-so-bad, and the actually-quite-pretty. Intensive Care Med 42, 2027-2029

31. Iba, T., Di Nisio, M., Thachil, J., Wada, H., Asakura, H., Sato, K., Kitamura, N., and Saitoh, D. (2016) Revision of the Japanese Association for Acute Medicine (JAAM) disseminated intravascular coagulation (DIC) diagnostic criteria using antithrombin activity. Crit Care 20, 287

32. Holvoet, P., Lijnen, H. R., and Collen, D. (1985) A monoclonal antibody specific for Lys-plasminogen. Application to the study of the activation pathways of plasminogen in vivo. J Biol Chem 260, 12106-12111

33. Grailhe, P., Nieuwenhuizen, W., and Angles-Cano, E. (1994) Study of tissue-type plasminogen activator binding sites on fibrin using distinct fragments of fibrinogen. Eur J Biochem 219, 961-967

34. Fleury, V., Loyau, S., Lijnen, H. R., Nieuwenhuizen, W., and Angles-Cano, E. (1993) Molecular assembly of plasminogen and tissue-type plasminogen activator on an evolving fibrin surface. Eur J Biochem 216, 549-556

35. Montes, R., Paramo, J. A., Angles-Cano, E., and Rocha, E. (1996) Development and clinical application of a new ELISA assay to determine plasmin-alpha2-antiplasmin complexes in plasma. Br J Haematol 92, 979-985

36. Grenier, A., Dehoux, M., Boutten, A., Arce-Vicioso, M., Durand, G., Gougerot-Pocidalo, M. A., and Chollet-Martin, S. (1999) Oncostatin M production and regulation by human polymorphonuclear neutrophils. Blood 93, 1413-1421

37. Angles-Cano, E., and Sultan, Y. (1984) A solid-phase fibrin immunoassay for the specific detection of monoclonal antibodies against different epitopic determinants of tissue-plasminogen activators. J Immunol Methods 69, 115-127

38. Dominguez, M., Montes, R., Paramo, J. A., and Angles-Cano, E. (2002) Bivalency of plasminogen monoclonal antibodies is required for plasminogen bridging to fibrin and enhanced plasmin formation. Biochim Biophys Acta 1598, 165-176

39. Koppert, P. W., Huijsmans, C. M., and Nieuwenhuizen, W. (1985) A monoclonal antibody, specific for human fibrinogen, fibrinopeptide A-containing fragments and not reacting with free fibrinopeptide A. Blood 66, 503-507

40. Rylatt, D. B., Blake, A. S., Cottis, L. E., Massingham, D. A., Fletcher, W. A., Masci, P. P., Whitaker, A. N., Elms, M., Bunce, I., Webber, A. J., and et al. (1983) An immunoassay for human D dimer using monoclonal antibodies. Thromb Res 31, 767-778

41. Bangert, K., and Thorsen, S. (2000) Assay of functional plasminogen in rat plasma applicable to experimental studies of thrombolysis. Thromb Haemost 84, 299-306

42. Pouit, L., Hudry-Clergeon, G., and Suscillon, M. (1973) Electron microscopy study of positive charges on fibrin fibres. Biochim Biophys Acta 317, 99-105

43. Campbell, E. J., and Owen, C. A. (2007) The sulfate groups of chondroitin sulfate- and heparan sulfate-containing proteoglycans in neutrophil plasma membranes are novel binding sites for human leukocyte elastase and cathepsin G. J Biol Chem 282, 14645-14654

44. Warejcka, D. J., and Twining, S. S. (2005) Specific conformational changes of plasminogen induced by chloride ions, 6-aminohexanoic acid and benzamidine, but not the overall openness of plasminogen regulate, production of biologically active angiostatins. Biochem J 392, 703-712

45. Massberg, S., Grahl, L., von Bruehl, M. L., Manukyan, D., Pfeiler, S., Goosmann, C., Brinkmann, V., Lorenz, M., Bidzhekov, K., Khandagale, A. B., Konrad, I., Kennerknecht, E., Reges, K., Holdenrieder, S., Braun, S., Reinhardt, C., Spannagl, M., Preissner, K. T., and Engelmann, B. (2010) Reciprocal coupling of coagulation and innate immunity via neutrophil serine proteases. Nat Med 16, 887-896

46. Clark, S. R., Ma, A. C., Tavener, S. A., McDonald, B., Goodarzi, Z., Kelly, M. M., Patel, K. D., Chakrabarti, S., McAvoy, E., Sinclair, G. D., Keys, E. M., Allen-Vercoe, E., Devinney, R., Doig, C. J., Green, F. H., and Kubes, P. (2007) Platelet TLR4 activates neutrophil extracellular traps to ensnare bacteria in septic blood. Nat Med 13, 463-469

47. McDonald, B., Davis, R. P., Kim, S. J., Tse, M., Esmon, C. T., Kolaczkowska, E., and Jenne, C. N. (2017) Platelets and neutrophil extracellular traps collaborate to promote intravascular coagulation during sepsis in mice. Blood 129, 1357-1367

48. Delabranche, X., Stiel, L., Severac, F., Galoisy, A. C., Mauvieux, L., Zobairi, F., Lavigne, T., Toti, F., Angles-Cano, E., Meziani, F., and Boisrame-Helms, J. (2017) Evidence of Netosis in Septic Shock-Induced Disseminated Intravascular Coagulation. Shock 47, 313-317

49. Maruchi, Y., Tsuda, M., Mori, H., Takenaka, N., Gocho, T., Huq, M. A., and Takeyama, N. (2018) Plasma myeloperoxidase-conjugated DNA level predicts outcomes and organ dysfunction in patients with septic shock. Crit Care 22, 176

50. Li, X., and Ma, X. (2018) A brief comment on the predictive value of myeloperoxidase-conjugated DNA level in patients with septic shock. Crit Care 22, 294

51. Kumar, S., Gupta, E., Kaushik, S., Srivastava, V. K., Saxena, J., Mehta, S., and Jyoti, A. (2019) Quantification of NETs formation in neutrophil and its correlation with the severity of sepsis and organ dysfunction. Clin Chim Acta 495, 606-610

52. Abrams, S. T., Morton, B., Alhamdi, Y., Alsabani, M., Lane, S., Welters, I. D., Wang, G., and Toh, C. H. (2019) A Novel Assay for Neutrophil Extracellular Traps (NETs) Formation Independently Predicts Disseminated Intravascular Coagulation and Mortality in Critically Ill Patients. Am J Respir Crit Care Med

53. Noubouossie, D. F., Reeves, B. N., Strahl, B. D., and Key, N. S. (2019) Neutrophils: back in the thrombosis spotlight. Blood 133, 2186-2197

54. Ducroux, C., Di Meglio, L., Loyau, S., Delbosc, S., Boisseau, W., Deschildre, C., Ben Maacha, M., Blanc, R., Redjem, H., Ciccio, G., Smajda, S., Fahed, R., Michel, J. B., Piotin, M., Salomon, L., Mazighi, M., Ho-Tin-Noe, B., and Desilles, J. P. (2018) Thrombus Neutrophil Extracellular Traps Content Impair tPA-Induced Thrombolysis in Acute Ischemic Stroke. Stroke 49, 754-757

55. Kolaczkowska, E., Jenne, C. N., Surewaard, B. G., Thanabalasuriar, A., Lee, W. Y., Sanz, M. J., Mowen, K., Opdenakker, G., and Kubes, P. (2015) Molecular mechanisms of NET formation and degradation revealed by intravital imaging in the liver vasculature. Nat Commun 6, 6673

56. Rouy, D., Koschinsky, M. L., Fleury, V., Chapman, J., and Angles-Cano, E. (1992) Apolipoprotein(a) and plasminogen interactions with fibrin: a study with recombinant apolipoprotein(a) and isolated plasminogen fragments. Biochemistry-Us 31, 6333-6339

57. Lamanuzzi, L. B., Mtairag el, M., Pepe, G., and Angles-Cano, E. (2004) Neutrophils stimulated by apolipoprotein(a) generate fragments that are stronger inhibitors of plasmin formation than apo(a). Thromb Haemost 92, 1066-1075

58. Thorsen, S., Clemmensen, I., Sottrup-Jensen, L., and Magnusson, S. (1981) Adsorption to fibrin of native fragments of known primary structure from human plasminogen. Biochim Biophys Acta 668, 377-387

59. Plow, E. F., and Edgington, T. S. (1975) "An alternative pathway for fibrinolysis. I. The cleavage of fibrinogen by leukocyte proteases at physiologic pH." J Clin Invest 56, 30-38

60. Machovich, R., and Owen, W. G. (1989) "An elastase-dependent pathway of plasminogen activation." Biochemistry-Us 28, 4517-4522

61. Wu, K., Urano, T., Ihara, H., Takada, Y., Fujie, M., Shikimori, M., Hashimoto, K., and Takada, A. (1995) "The cleavage and inactivation of plasminogen activator inhibitor type1 by neutrophil elastase: the evaluation of its physiologic relevance in fibrinolysis." Blood 86, 1056-1061

62. Duboscq, C., Genoud, V., Parborell, M. F., and Kordich, L. C. (1997) Impaired clot lysis by rt-PA catalyzed mini-plasminogen activation. Thromb Res 86, 505-513

63. Suenson, E., Lutzen, O., and Thorsen, S. (1984) Initial plasmin-degradation of fibrin as the basis of a positive feedback mechanism in fibrinolysis. Eur J Biochem 140, 513-522

64. Herren, T., Burke, T. A., Das, R., and Plow, E. F. (2006) Identification of histone H2B as a regulated plasminogen receptor. Biochemistry-Us 45, 9463-9474

65. Duboscq, C., Quintana, I., Bassilotta, E., Bergonzelli, G. E., Porterie, P., Sassetti, B., Haedo, A. S., Wainsztein, N., Kruithof, E. K., and Kordich, L. (1997) Plasminogen: an important hemostatic parameter in septic patients. Thromb Haemost 77, 1090-1095

66. Lamanuzzi, L. B., Mtairag el, M., Pepe, G., and Angles-Cano, E. (2004) Neutrophils stimulated by apolipoprotein(a) generate fragments that are stronger inhibitors of plasmin formation than apo(a). Thromb Haemost 92, 1066-1075

67. Shevchenko, A., Loboda, A., Ens, W., Schraven, B., Standing, K. G., and Shevchenko, A. (2001) Archived polyacrylamide gels as a resource for proteome characterization by mass spectrometry. Electrophoresis 22, 1194-1203

68 Warejcka, D. J., and Twining, S. S. (2005) Specific conformational changes of plasminogen induced by chloride ions, 6-aminohexanoic acid and benzamidine, but not the overall openness of plasminogen regulate, production of biologically active angiostatins. Biochem J 392, 703-712

69 "Disseminated Intravascular Coagulation | NHLBI, NIH". www.nhlbi.nih.gov. Retrieved 20 December 2017.

70 Levi M, Scully M How I treat Disseminated Intravascular coagulation Blood 2018; 131 (8), 845-854.

71 Podolska MJ, et al. Autoimmunity 2018 - Review. PMID 30369262.

72 Cancer Immunol Immunother 2020 PMID 31982939.

73 Breast Cancer Res 2019 PMID 31852512

74. Neutrophil Extracellular Traps Associate With Clinical Stages in Breast Cancer Science Translational Medicine Pathol Oncol Res 19 Oct 2016:Vol. 8, Issue 361, pp. 361ra138

75. Law et al. Cell Reports| Volume 1, ISSUE 3, P185-190, March 29, 2012).

76. Lijnen et al 2001 Enzyme-linked Immunosorbent Assay for the Specific Detection of Angiostatin-Like Plasminogen Moieties in Biological Samples Thromb Res 2001 102 (1), 53-9.

77. Criteria for specific measurement of plasminogen (enzymatic; procedure) in human plasma", eJIFCC, vol 12 No 2, 2000

78. M Sten-Linder, C Linder, H Strander, E Munck-Wikland, P Wersäll, S Linder, B Wiman "Angiostatin Fragments in Urine From Patients With Malignant Disease" Anticancer Res 1999; 19 ;3409-14

79 Y Takada "Potential Role of Kringle-Integrin Interaction in Plasmin and uPA" Actions Journal of Biomedicine and Biotechnology Volume 2012, Article ID 136302, 8 pages doi:10.1155/2012/136302

80. Z Zhang "Plasminogen Kringle 5 Inhibits Alkali-Burn-Induced Corneal Investigative" Ophthalmology & Visual Science November 2005, Vol.46, 4062-4071. doi:https://doi.org/10.1167/iovs.04-1330

81. Barbosa da Cruz D et al "DNA-bound Elastase of Neutrophil Extracellular Traps Degrades Plasminogen, Reduces Plasmin Formation, and Decreases Fibrinolysis: Proof of Concept in Septic Shock Plasma" FASEB J 20189,33,14270)

82. Constantinescu et al. "Amorphous protein aggregates stimulate plasminogen activation, leading to release of cytotoxic fragments that are clients for extracellular chaperones." J. Biol. Chem. (2017) 292(35) 14425-14437

83. Rife U, Milgrom F, Shulman S. Antigenic analysis of plasminogen and plasmin. Blood 1963,212,322 Okamoto A et al. Population based distribution of plasminogen... J Thromb Haemost 2003,1,2397

## Claims

1. An in-vitro process for predicting and/or detecting fibrinolytic insufficiency associated to pathologies with NETs (Neutrophil Extracellular Traps) with/without disseminated intravascular coagulation (DIC) from a first biological sample comprising measuring the concentration of Plasminogen and at least one fragment thereof.

2. The process according to claim 1 wherein pathologies are infectious or non-infectious

3. The process according to claim 1 or 2 wherein pathologies associated with NETs are selected from the group comprising sepsis, septic shock, ischemic stroke, coronary thrombosis, cancer, trauma and autoimmune diseases.

4. The process according to in any one of claims 1 to 3, wherein the biological sample is selected from the group comprising a blood sample and plasma sample.

5. The process for predicting and/or detecting fibrinolytic insufficiency, according to any one of claim 1 to 4, wherein said plasminogen fragment is selected from the group of fragment comprising kringle (K) 1 to 3 domains ($K_{1+2+3}$), kringle (K) 1 to 4 domains ($K_{1+2+3+4}$) and kringle (K) 5 domain and the serine protease (SP) region (mini-plasminogen) ($K_5$-SP).

6. The process for predicting and/or detecting fibrinolytic insufficiency, according to any one of claim 1 to 5, wherein the biological sample originate from a patient with septic shock.

7. The process for predicting and/or detecting pathology associated with NETs, according to any one of claim 1 to 6, comprising measuring the concentration of mini-plasminogen (K5-SP).

8. An in-vitro process for determining the efficacy of a treatment of fibrinolytic insufficiency associated to pathologies with NETs with/without disseminated intravascular coagulation (DIC) comprising :

   a. Determination and/or measurement of the concentration C1 of Plasminogen and fragment thereof from a first biological sample before treatment with a compound,
   b. Determination and/or measurement of the concentration C2 of Plasminogen and fragment thereof from a second biological sample after treatment with said compound, and optionally
   c. comparison of the concentrations and calculation of a score (S) according to the following formula:

$$S = C2/C1,$$

   a value of S greater than 1 indicating that the treatment is effective.

9. The process according to claim 8 , wherein said first and second biological samples originate from a patient with septic shock.

**Patentansprüche**

1. In-vitro-Verfahren zur Vorhersage und/oder zum Nachweis einer fibrinolytischen Insuffizienz, die mit Pathologien mit NETs mit/ohne disseminierte intravaskuläre Koagulation (DIC) verbunden ist, aus einer ersten biologischen Probe, umfassend die Messung der Konzentration von Plasminogen und mindestens einem Fragment davon

2. Verfahren nach Anspruch 1, wobei die Pathologien infektiös oder nicht-infektiös sind

3. Verfahren nach Anspruch 1 oder 2, wobei mit NETs assoziierte Pathologien aus der Gruppe ausgewählt werden, die Sepsis, septischen Schock, ischämischen Schlaganfall, koronare Thrombose, Krebs, Trauma und Autoimmunerkrankungen umfasst.

4. Verfahren nach einem der Ansprüche 1 bis 3, wobei die biologische Probe aus der Gruppe ausgewählt wird, die eine Blutprobe und eine Plasmaprobe umfasst.

5. Verfahren zur Vorhersage und/oder zum Nachweis einer fibrinolytischen Insuffizienz nach einem der Ansprüche 1 bis 4, wobei das Plasminogenfragment aus der Gruppe von Fragmenten ausgewählt ist, die Kringle (K) 1 bis 3 Domänen $(K_{1+2+3})$, Kringle (K) 1 bis 4 Domänen $(K_{1+2+3+4})$ und Kringle (K) 5 Domäne und die Serinprotease (SP) Region (Mini-Plasminogen) $(K_5$-SP) umfassen.

6. Verfahren zur Vorhersage und/oder zum Nachweis einer fibrinolytischen Insuffizienz nach einem der Ansprüche 1 bis 5, wobei die biologische Probe von einem Patienten mit septischem Schock stammt.

7. Verfahren zur Vorhersage und/oder zum Nachweis der mit NETs verbundenen Pathologie nach einem der Ansprüche 1 bis 6, umfassend die Messung der Konzentration von Mini-Plasminogen (K5-SP).

8. In-vitro-Verfahren zur Bestimmung der Wirksamkeit einer Behandlung von fibrinolytischer Insuffizienz, die mit Pathologien mit NETs mit/ohne disseminierte intravaskuläre Gerinnung (DIC) verbunden ist, umfassend:

    a. Bestimmung und/oder Messung der Konzentration C1 von Plasminogen und dessen Fragment aus einer ersten biologischen Probe vor der Behandlung mit einer Verbindung,
    b. Bestimmung und/oder Messung der Konzentration C2 von Plasminogen und dessen Fragment aus einer zweiten biologischen Probe nach Behandlung mit der Verbindung, und gegebenenfalls
    c. Vergleich der Konzentrationen und Berechnung einer Punktzahl (S) nach der folgenden Formel:

$$S = C2/C1,$$

ein Wert von S größer als 1 zeigt an, dass die Behandlung wirksam ist.

9. Verfahren nach Anspruch 8, wobei die erste und zweite biologische Probe von einem Patienten mit septischem Schock stammen.

**Revendications**

1. Procédé in-vitro pour prédire et/ou détecter une insuffisance fibrinolytique associée à des pathologies avec des NETs (pièges extracellulaires des neutrophiles) avec/sans coagulation intravasculaire disséminée (CIVD) à partir d'un premier échantillon biologique comprenant la mesure de la concentration de plasminogène et d'au moins un de ses fragments.

2. Procédé selon la revendication 1, dans lequel les pathologies sont infectieuses ou non infectieuses.

3. Procédé selon la revendication 1 ou 2, dans lequel les pathologies associées aux NETs sont choisies dans le groupe comprenant la septicémie, le choc septique, l'accident vasculaire cérébral ischémique, la thrombose coronarienne, le cancer, le traumatisme et les maladies auto-immunes.

4. Procédé selon l'une des revendications 1 à 3, dans lequel l'échantillon biologique est choisi dans le groupe comprenant un échantillon de sang et un échantillon de plasma.

5. Procédé de prédiction et/ou de détection de l'insuffisance fibrinolytique, selon l'une des revendications 1 à 4, dans lequel ledit fragment de plasminogène est choisi dans le groupe des fragments comprenant les domaines kringle (K) 1 à 3 ($K_{1+2+3}$), les domaines kringle (K) 1 à 4 ($K_{1+2+3+4}$) et le domaine kringle (K) 5 et la région sérine protéase (SP) (mini-plasminogène) ($K_5$-SP).

6. Procédé de prédiction et/ou de détection de l'insuffisance fibrinolytique, selon l'une des revendications 1 à 5, dans lequel l'échantillon biologique provient d'un patient en état de choc septique.

7. Procédé de prédiction et/ou de détection de la pathologie associée aux NETs, selon l'une des revendications 1 à 6, comprenant la mesure de la concentration du mini-plasminogène (K5-SP).

8. Procédé in-vitro pour déterminer l'efficacité d'un traitement de l'insuffisance fibrinolytique associée à des pathologies avec des NETs avec/sans coagulation intravasculaire disséminée (CIVD) comprenant :

   a. Détermination et/ou mesure de la concentration C1 de plasminogène et de ses fragments dans un premier échantillon biologique avant traitement par un composé,
   b. Détermination et/ou mesure de la concentration C2 du plasminogène et de ses fragments dans un deuxième échantillon biologique après traitement par ledit composé, et éventuellement
   c. comparaison des concentrations et calcul d'un score (S) selon la formule suivante :

$$S = C2/C1,$$

une valeur de S supérieure à 1 indiquant que le traitement est efficace.

9. Procédé selon la revendication 8, dans lequel les premier et deuxième échantillons biologiques proviennent d'un patient en état de choc septique.

FIGURE 1

FIGURE 2

FIGURE 3

FIGURE 4

FIGURE 5

FIGURE 6

**FIGURE 7**

**FIGURE 8**

**FIGURE 9**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 1985004425 A

**Non-patent literature cited in the description**

- **LAW et al.** *Cell Reports*, 29 March 2012, vol. 1 (3), 185-190
- Criteria for specific measurement of plasminogen (enzymatic; procedure) in human plasma. *eJIFCC*, 2000, vol. 12 (2)
- **MASSBERG, S. et al.** Reciprocal coupling of coagulation and innate immunity via neutrophil serine proteases. *Nat Med*, vol. 16, 887-896
- **RIFE U ; MILGROM F ; SHULMAN S**. Antigenic analysis of plasminogen and plasmin. *Blood*, 1963, vol. 212, 322
- **OKAMOTO A et al.** Population based distribution of plasminogen.... *J Thromb Haemost*, 2003, vol. 2397 (83)
- **LLJNEN et al.** Enzyme-linked Immunosorbent Assay for the Specific Detection of Angiostatin-Like Plasminogen Moieties in Biological Samples. *Thromb Res*, 2001, vol. 102 (1), 53-9
- **M STEN-LINDER et al.** Angiostatin Fragments in Urine From Patients With Malignant Disease. *Anticancer Res*, 1999, vol. 19, 3409-14
- **Y TAKADA**. Potential Role of Kringle-Integrin Interaction in Plasmin and uPA. *Actions Journal of Biomedicine and Biotechnology*, vol. 2012, 8
- **Z ZHANG**. Plasminogen Kringle 5 Inhibits Alkali-Burn-Induced Corneal Investigative. *Ophthalmology & Visual Science*, November 2005, vol. 46, 4062-4071
- **ROUY D et al.** Apolipoprotein(a) and Plasminogen Interactions With Fibrin: A Study With Recombinant Apolipoprotein(a) and Isolated Plasminogen Fragments. *Biochemistry*, 1992, vol. 6333 (56)
- **BARBOSA DA CRUZ D et al.** DNA-bound Elastase of Neutrophil Extracellular Traps Degrades Plasminogen, Reduces Plasmin Formation, and Decreases Fibrinolysis: Proof of Concept in Septic Shock Plasma. *FASEB J*, vol. 33, 14270
- **CONSTANTINESCU et al.** Amorphous protein aggregates stimulate plasminogen activation, leading to release of cytotoxic fragments that are clients for extracellular chaperones. *J. Biol. Chem.*, 2017, vol. 292 (35), 14425-14437
- **LIJNEN et al.** Enzyme-linked Immunosorbent Assay for the Specific Detection of Angiostatin-Like Plasminogen Moieties in Biological Samples. *Thromb Res*, 2001, vol. 102 (1), 53-9
- **MOROZ et al.** Mini-plasminogen-likfer agments of plasminogen in synovial fluid in acute inflammatory arthritis. *Thromb Res*, 1986, vol. 43, 417
- *Biochem J*, 1995
- *Biochemistry*, 1991
- *Thromb Haemostas*, 1991
- *J Lab Clin Med*, 1992
- *Circulation*, 1995
- **LONGSTAFF, C. ; KOLEV, K.** Basic mechanisms and regulation of fibrinolysis. *J Thromb Haemost*, 2015, vol. 1, 98-105
- **XUE, Y. ; BODIN, C ; OLSSON, K.** Crystal structure of the native plasminogen reveals an activation-resistant compact conformation. *J Thromb Haemost*, 2012, vol. 10, 1385-1396
- **FLEURY, V. ; ANGLES-CANO, E.** Characterization of the binding of plasminogen to fibrin surfaces: the role of carboxy-terminal lysines. *Biochemistry-Us*, 1991, vol. 30, 7630-7638
- **SCAPINI, P. ; NESI, L. ; MORINI, M. ; TANGHETTI, E. ; BELLERI, M. ; NOONAN, D. ; PRESTA, M. ; ALBINI, A. ; CASSATELLA, M. A.** Generation of biologically active angiostatin kringle 1-3 by activated human neutrophils. *J Immunol*, 2002, vol. 168, 5798-5804
- **MOROZ, L. A.** Mini-plasminogen: a mechanism for leukocyte modulation of plasminogen activation by urokinase. *Blood*, 1981, vol. 58, 97-104
- **BEATTY, K. ; BIETH, J. ; TRAVIS, J.** Kinetics of association of serine proteinases with native and oxidized alpha-1-proteinase inhibitor and alpha-1-antichymotrypsin. *J Biol Chem*, 1980, vol. 255, 3931-3934
- **KORKMAZ, B. ; ATTUCCI, S. ; JOURDAN, M. L. ; JULIANO, L. ; GAUTHIER, F.** Inhibition of neutrophil elastase by alpha1-protease inhibitor at the surface of human polymorphonuclear neutrophils. *J Immunol*, 2005, vol. 175, 3329-3338

- **OWEN, C. A.** ; **CAMPBELL, M. A.** ; **SANNES, P. L.** ; **BOUKEDES, S. S.** ; **CAMPBELL, E. J.** Cell surface-bound elastase and cathepsin G on human neutrophils: a novel, non-oxidative mechanism by which neutrophils focus and preserve catalytic activity of serine proteinases. *J Cell Biol*, 1995, vol. 131, 775-789
- **BELORGEY, D.** ; **BIETH, J. G.** Effect of polynucleotides on the inhibition of neutrophil elastase by mucus proteinase inhibitor and alpha 1-proteinase inhibitor. *Biochemistry-Us*, 1998, vol. 37, 16416-16422
- **BELORGEY, D.** ; **BIETH, J. G.** DNA binds neutrophil elastase and mucus proteinase inhibitor and impairs their functional activity. *FEBS Lett*, 1995, vol. 361, 265-268
- **DURANTON, J.** ; **BELORGEY, D.** ; **CARRERE, J.** ; **DONATO, L.** ; **MORITZ, T.** ; **BIETH, J. G.** Effect of DNase on the activity of neutrophil elastase, cathepsin G and proteinase 3 in the presence of DNA. *FEBS Lett*, 2000, vol. 473, 154-156
- **DURANTON, J.** ; **BOUDIER, C.** ; **BELORGEY, D.** ; **MELLET, P.** ; **BIETH, J. G.** DNA strongly impairs the inhibition of cathepsin G by alpha(1)-antichymotrypsin and alpha(1)-proteinase inhibitor. *J Biol Chem*, 2000, vol. 275, 3787-3792
- **BRINKMANN, V.** ; **REICHARD, U.** ; **GOOSMANN, C.** ; **FAULER, B.** ; **UHLEMANN, Y.** ; **WEISS, D. S.** ; **WEINRAUCH, Y.** ; **ZYCHLINSKY, A.** Neutrophil extracellular traps kill bacteria. *Science*, 2004, vol. 303, 1532-1535
- **KENNY, E. F.** ; **HERZIG, A.** ; **KRUGER, R.** ; **MUTH, A.** ; **MONDAL, S.** ; **THOMPSON, P. R.** ; **BRINKMANN, V.** ; **BERNUTH, H. V.** ; **ZYCHLINSKY, A.** Diverse stimuli engage different neutrophil extracellular trap pathways. *Elife*, 2017, vol. 6
- **FUCHS, T. A.** ; **ABED, U.** ; **GOOSMANN, C.** ; **HURWITZ, R.** ; **SCHULZE, I.** ; **WAHN, V.** ; **WEINRAUCH, Y.** ; **BRINKMANN, V.** ; **ZYCHLINSKY, A.** Novel cell death program leads to neutrophil extracellular traps. *J Cell Biol*, 2007, vol. 176, 231-241
- **URBAN, C. F.** ; **ERMERT, D.** ; **SCHMID, M.** ; **ABU-ABED, U.** ; **GOOSMANN, C.** ; **NACKEN, W.** ; **BRINKMANN, V.** ; **JUNGBLUT, P. R.** ; **ZYCHLINSKY, A.** Neutrophil extracellular traps contain calprotectin, a cytosolic protein complex involved in host defense against Candida albicans. *Plos Pathog*, 2009, vol. 5, e1000639
- **THOMAS, M. P.** ; **WHANGBO, J.** ; **MCCROSSAN, G.** ; **DEUTSCH, A. J.** ; **MARTINOD, K.** ; **WALCH, M.** ; **LIEBERMAN, J.** Leukocyte protease binding to nucleic acids promotes nuclear localization and cleavage of nucleic acid binding proteins. *J Immunol*, 2014, vol. 192, 5390-5397
- **BRINKMANN, V.** ; **ZYCHLINSKY, A.** Beneficial suicide: why neutrophils die to make NETs. *Nat Rev Microbiol*, 2007, vol. 5, 577-582
- **KESSENBROCK, K.** ; **KRUMBHOLZ, M.** ; **SCHONERMARCK, U.** ; **BACK, W.** ; **GROSS, W. L.** ; **WERB, Z.** ; **GRONE, H. J.** ; **BRINKMANN, V.** ; **JENNE, D. E.** Netting neutrophils in autoimmune small-vessel vasculitis. *Nat Med*, 2009, vol. 15, 623-625
- **ENGELMANN, B.** ; **MASSBERG, S.** Thrombosis as an intravascular effector of innate immunity. *Nat Rev Immunol*, 2013, vol. 13, 34-45
- **FUCHS, T. A.** ; **BRILL, A.** ; **DUERSCHMIED, D.** ; **SCHATZBERG, D.** ; **MONESTIER, M.** ; **MYERS, D. D., JR.** ; **WROBLESKI, S. K.** ; **WAKEFIELD, T. W.** ; **HARTWIG, J. H.** ; **WAGNER, D. D.** Extracellular DNA traps promote thrombosis. *Proc Natl Acad Sci U S A*, 2010, vol. 107, 15880-15885
- **GRASSLE, S.** ; **HUCK, V.** ; **PAPPELBAUM, K. I.** ; **GORZELANNY, C.** ; **APONTE-SANTAMARIA, C.** ; **BALDAUF, C., GRATER, F.** ; **SCHNEPPENHEIM, R.** ; **OBSER, T.** ; **SCHNEIDER, S. W.** von Willebrand factor directly interacts with DNA from neutrophil extracellular traps. *Arterioscler Thromb Vasc Biol*, 2014, vol. 34, 1382-1389
- **DE BOER, O. J.** ; **LI, X.** ; **TEELING, P.** ; **MACKAAY, C.** ; **PLOEGMAKERS, H. J.** ; **VAN DER LOOS, C. M.** ; **DAEMEN, M. J.** ; **DE WINTER, R. J.** ; **VAN DER WAL, A. C.** Neutrophils, neutrophil extracellular traps and interleukin-17 associate with the organisation of thrombi in acute myocardial infarction. *Thromb Haemost*, 2013, vol. 109, 290-297
- **RIEGGER, J.** ; **BYRNE, R. A.** ; **JONER, M.** ; **CHANDRARATNE, S.** ; **GERSHLICK, A. H.** ; **TEN BERG, J. M.** ; **ADRIAENSSENS, T.** ; **GUAGLIUMI, G.** ; **GODSCHALK, T. C.** ; **NEUMANN, F. J.** Histopathological evaluation of thrombus in patients presenting with stent thrombosis. A multicenter European study: a report of the prevention of late stent thrombosis by an interdisciplinary global European effort consortium. *Eur Heart J*, 2016, vol. 37, 1538-1549
- **LARIDAN, E.** ; **DENORME, F.** ; **DESENDER, L.** ; **FRANCOIS, O.** ; **ANDERSSON, T.** ; **DECKMYN, H.** ; **VANHOORELBEKE, K.** ; **DE MEYER, S. F.** Neutrophil extracellular traps in ischemic stroke thrombi. *Ann Neurol*, 2017, vol. 82, 223-232
- **LONGSTAFF, C.** ; **VARJU, I.** ; **SOTONYI, P.** ; **SZABO, L.** ; **KRUMREY, M.** ; **HOELL, A.** ; **BOTA, A.** ; **VARGA, Z.** ; **KOMOROWICZ, E.** ; **KOLEV, K.** Mechanical stability and fibrinolytic resistance of clots containing fibrin, DNA, and histones. *J Biol Chem*, 2013, vol. 288, 6946-6956
- **GOULD, T. J.** ; **VU, T. T.** ; **STAFFORD, A. R.** ; **DWIVEDI, D. J.** ; **KIM, P. Y.** ; **FOX-ROBICHAUD, A. E.** ; **WEITZ, J. I.** ; **LIAW, P. C.** Cell-Free DNA Modulates Clot Structure and Impairs Fibrinolysis in Sepsis. *Arterioscler Thromb Vasc Biol*, 2015, vol. 35, 2544-2553

- **VARJU, I.** ; **LONGSTAFF, C.** ; **SZABO, L.** ; **FARKAS, A. Z.** ; **VARGA-SZABO, V. J.** ; **TANKA-SALAMON, A.** ; **MACHOVICH, R.** ; **KOLEV, K.** DNA, histones and neutrophil extracellular traps exert anti-fibrinolytic effects in a plasma environment. *Thromb Haemost*, 2015, vol. 113, 1289-1298
- **FUCHS, T. A.** ; **BRILL, A.** ; **WAGNER, D. D.** Neutrophil extracellular trap (NET) impact on deep vein thrombosis. *Arterioscler Thromb Vasc Biol*, 2012, vol. 32, 1777-1783
- **SINGER, M.** The new sepsis consensus definitions (Sepsis-3): the good, the not-so-bad, and the actually-quite-pretty. *Intensive Care Med*, 2016, vol. 42, 2027-2029
- **IBA, T.** ; **DI NISIO, M.** ; **THACHIL, J.** ; **WADA, H.** ; **ASAKURA, H.** ; **SATO, K.** ; **KITAMURA, N.** ; **SAITOH, D.** Revision of the Japanese Association for Acute Medicine (JAAM) disseminated intravascular coagulation (DIC) diagnostic criteria using antithrombin activity. *Crit Care*, 2016, vol. 20, 287
- **HOLVOET, P.** ; **LIJNEN, H. R.** ; **COLLEN, D.** A monoclonal antibody specific for Lys-plasminogen. Application to the study of the activation pathways of plasminogen in vivo. *J Biol Chem*, 1985, vol. 260, 12106-12111
- **GRAILHE, P.** ; **NIEUWENHUIZEN, W.** ; **ANGLES-CANO, E.** Study of tissue-type plasminogen activator binding sites on fibrin using distinct fragments of fibrinogen. *Eur J Biochem*, 1994, vol. 219, 961-967
- **FLEURY, V.** ; **LOYAU, S.** ; **LIJNEN, H. R.** ; **NIEUWENHUIZEN, W.** ; **ANGLES-CANO, E.** Molecular assembly of plasminogen and tissue-type plasminogen activator on an evolving fibrin surface. *Eur J Biochem*, 1993, vol. 216, 549-556
- **MONTES, R.** ; **PARAMO, J. A.** ; **ANGLES-CANO, E.** ; **ROCHA, E.** Development and clinical application of a new ELISA assay to determine plasmin-alpha2-antiplasmin complexes in plasma. *Br J Haematol*, 1996, vol. 92, 979-985
- **GRENIER, A.** ; **DEHOUX, M.** ; **BOUTTEN, A.** ; **ARCE-VICIOSO, M.** ; **DURAND, G.** ; **GOUGEROT-POCIDALO, M. A.** ; **CHOLLET-MARTIN, S.** Oncostatin M production and regulation by human polymorphonuclear neutrophils. *Blood*, 1999, vol. 93, 1413-1421
- **ANGLES-CANO, E.** ; **SULTAN, Y.** A solid-phase fibrin immunoassay for the specific detection of monoclonal antibodies against different epitopic determinants of tissue-plasminogen activators. *J Immunol Methods*, 1984, vol. 69, 115-127
- **DOMINGUEZ, M.** ; **MONTES, R.** ; **PARAMO, J. A.** ; **ANGLES-CANO, E.** Bivalency of plasminogen monoclonal antibodies is required for plasminogen bridging to fibrin and enhanced plasmin formation. *Biochim Biophys Acta*, 2002, vol. 1598, 165-176
- **KOPPERT, P. W.** ; **HUIJSMANS, C. M.** ; **NIEUWENHUIZEN, W.** A monoclonal antibody, specific for human fibrinogen, fibrinopeptide A-containing fragments and not reacting with free fibrinopeptide A. *Blood*, 1985, vol. 66, 503-507
- **RYLATT, D. B.** ; **BLAKE, A. S.** ; **COTTIS, L. E.** ; **MASSINGHAM, D. A.** ; **FLETCHER, W. A.** ; **MASCI, P. P.** ; **WHITAKER, A. N.** ; **ELMS, M.** ; **BUNCE, I.** ; **WEBBER, A. J. et al.** An immunoassay for human D dimer using monoclonal antibodies. *Thromb Res*, 1983, vol. 31, 767-778
- **BANGERT, K.** ; **THORSEN, S.** Assay of functional plasminogen in rat plasma applicable to experimental studies of thrombolysis. *Thromb Haemost*, 2000, vol. 84, 299-306
- **POUIT, L.** ; **HUDRY-CLERGEON, G.** ; **SUSCILLON, M.** Electron microscopy study of positive charges on fibrin fibres. *Biochim Biophys Acta*, 1973, vol. 317, 99-105
- **CAMPBELL, E. J.** ; **OWEN, C. A.** The sulfate groups of chondroitin sulfate- and heparan sulfate-containing proteoglycans in neutrophil plasma membranes are novel binding sites for human leukocyte elastase and cathepsin G. *J Biol Chem*, 2007, vol. 282, 14645-14654
- **WAREJCKA, D. J.** ; **TWINING, S. S.** Specific conformational changes of plasminogen induced by chloride ions, 6-aminohexanoic acid and benzamidine, but not the overall openness of plasminogen regulate, production of biologically active angiostatins. *Biochem J*, 2005, vol. 392, 703-712
- **MASSBERG, S.** ; **GRAHL, L.** ; **VON BRUEHL, M. L.** ; **MANUKYAN, D.** ; **PFEILER, S.** ; **GOOSMANN, C.** ; **BRINKMANN, V.** ; **LORENZ, M.** ; **BIDZHEKOV, K.** ; **KHANDAGALE, A. B.** Reciprocal coupling of coagulation and innate immunity via neutrophil serine proteases. *Nat Med*, 2010, vol. 16, 887-896
- **CLARK, S. R.** ; **MA, A. C.** ; **TAVENER, S. A.** ; **MCDONALD, B.** ; **GOODARZI, Z.** ; **KELLY, M. M.** ; **PATEL, K. D.** ; **CHAKRABARTI, S.** ; **MCAVOY, E.** ; **SINCLAIR, G. D.** Platelet TLR4 activates neutrophil extracellular traps to ensnare bacteria in septic blood. *Nat Med*, 2007, vol. 13, 463-469
- **MCDONALD, B.** ; **DAVIS, R. P.** ; **KIM, S. J.** ; **TSE, M.** ; **ESMON, C. T.** ; **KOLACZKOWSKA, E.** ; **JENNE, C. N.** Platelets and neutrophil extracellular traps collaborate to promote intravascular coagulation during sepsis in mice. *Blood*, 2017, vol. 129, 1357-1367
- **DELABRANCHE, X.** ; **STIEL, L.** ; **SEVERAC, F.** ; **GALOISY, A. C.** ; **MAUVIEUX, L.** ; **ZOBAIRI, F.** ; **LAVIGNE, T.** ; **TOTI, F.** ; **ANGLES-CANO, E.** ; **MEZIANI, F.** Evidence of Netosis in Septic Shock-Induced Disseminated Intravascular Coagulation. *Shock*, 2017, vol. 47, 313-317

- **MARUCHI, Y.** ; **TSUDA, M.** ; **MORI, H.** ; **TAKENAKA, N.** ; **GOCHO, T.** ; **HUQ, M. A.** ; **TAKEYAMA, N.** Plasma myeloperoxidase-conjugated DNA level predicts outcomes and organ dysfunction in patients with septic shock. *Crit Care*, 2018, vol. 22, 176

- **LI, X.** ; **MA, X.** A brief comment on the predictive value of myeloperoxidase-conjugated DNA level in patients with septic shock. *Crit Care*, 2018, vol. 22, 294

- **KUMAR, S.** ; **GUPTA, E.** ; **KAUSHIK, S.** ; **SRIVASTAVA, V. K.** ; **SAXENA, J.** ; **MEHTA, S.** ; **JYOTI, A.** Quantification of NETs formation in neutrophil and its correlation with the severity of sepsis and organ dysfunction. *Clin Chim Acta*, 2019, vol. 495, 606-610

- **ABRAMS, S. T.** ; **MORTON, B.** ; **ALHAMDI, Y.** ; **ALSABANI, M.** ; **LANE, S.** ; **WELTERS, I. D.** ; **WANG, G.** ; **TOH, C. H.** A Novel Assay for Neutrophil Extracellular Traps (NETs) Formation Independently Predicts Disseminated Intravascular Coagulation and Mortality in Critically III Patients. *Am J Respir Crit Care Med*, 2019

- **NOUBOUOSSIE, D. F.** ; **REEVES, B. N.** ; **STRAHL, B. D.** ; **KEY, N. S.** Neutrophils: back in the thrombosis spotlight. *Blood*, 2019, vol. 133, 2186-2197

- **DUCROUX, C.** ; **DI MEGLIO, L.** ; **LOYAU, S.** ; **DELBOSC, S.** ; **BOISSEAU, W.** ; **DESCHILDRE, C.** ; **BEN MAACHA, M.** ; **BLANC, R.** ; **REDJEM, H.** ; **CICCIO, G.** Thrombus Neutrophil Extracellular Traps Content Impair tPA-Induced Thrombolysis in Acute Ischemic Stroke. *Stroke*, 2018, vol. 49, 754-757

- **KOLACZKOWSKA, E.** ; **JENNE, C. N.** ; **SUREWAARD, B. G.** ; **THANABALASURIAR, A.** ; **LEE, W. Y.** ; **SANZ, M. J.** ; **MOWEN, K.** ; **OPDENAKKER, G.** ; **KUBES, P.** Molecular mechanisms of NET formation and degradation revealed by intravital imaging in the liver vasculature. *Nat Commun*, 2015, vol. 6, 6673

- **ROUY, D.** ; **KOSCHINSKY, M. L.** ; **FLEURY, V.** ; **CHAPMAN, J.** ; **ANGLES-CANO, E.** Apolipoprotein(a) and plasminogen interactions with fibrin: a study with recombinant apolipoprotein(a) and isolated plasminogen fragments. *Biochemistry-Us*, 1992, vol. 31, 6333-6339

- **LAMANUZZI, L. B.** ; **MTAIRAG EL, M.** ; **PEPE, G.** ; **ANGLES-CANO, E.** Neutrophils stimulated by apolipoprotein(a) generate fragments that are stronger inhibitors of plasmin formation than apo(a). *Thromb Haemost*, 2004, vol. 92, 1065-1075

- **THORSEN, S.** ; **CLEMMENSEN, I.** ; **SOTTRUP-JENSEN, L.** ; **MAGNUSSON, S.** Adsorption to fibrin of native fragments of known primary structure from human plasminogen. *Biochim Biophys Acta*, 1981, vol. 668, 377-387

- **PLOW, E. F.** ; **EDGINGTON, T. S.** An alternative pathway for fibrinolysis. I. The cleavage of fibrinogen by leukocyte proteases at physiologic pH. *J Clin Invest*, 1975, vol. 56, 30-38

- **MACHOVICH, R.** ; **OWEN, W. G.** An elastase-dependent pathway of plasminogen activation. *Biochemistry-Us*, 1989, vol. 28, 4517-4522

- **WU, K.** ; **URANO, T.** ; **IHARA, H.** ; **TAKADA, Y.** ; **FUJIE, M.** ; **SHIKIMORI, M.** ; **HASHIMOTO, K.** ; **TAKADA, A.** The cleavage and inactivation of plasminogen activator inhibitor type1 by neutrophil elastase: the evaluation of its physiologic relevance in fibrinolysis. *Blood*, 1995, vol. 86, 1056-1061

- **DUBOSCQ, C.** ; **GENOUD, V.** ; **PARBORELL, M. F.** ; **KORDICH, L. C.** Impaired clot lysis by rt-PA catalyzed mini-plasminogen activation. *Thromb Res*, 1997, vol. 86, 505-513

- **SUENSON, E.** ; **LUTZEN, O.** ; **THORSEN, S.** Initial plasmin-degradation of fibrin as the basis of a positive feed-back mechanism in fibrinolysis. *Eur J Biochem*, 1984, vol. 140, 513-522

- **HERREN, T.** ; **BURKE, T. A.** ; **DAS, R.** ; **PLOW, E. F.** Identification of histone H2B as a regulated plasminogen receptor. *Biochemistry-Us*, 2006, vol. 45, 9463-9474

- **DUBOSCQ, C.** ; **QUINTANA, I.** ; **BASSILOTTA, E.** ; **BERGONZELLI, G. E.** ; **PORTERIE, P.** ; **SASSETTI, B.** ; **HAEDO, A. S.** ; **WAINSZTEIN, N.** ; **KRUITHOF, E. K.** ; **KORDICH, L.** Plasminogen: an important hemostatic parameter in septic patients. *Thromb Haemost*, 1997, vol. 77, 1090-1095

- **LAMANUZZI, L. B.** ; **MTAIRAG EL, M.** ; **PEPE, G.** ; **ANGLES-CANO, E.** Neutrophils stimulated by apolipoprotein(a) generate fragments that are stronger inhibitors of plasmin formation than apo(a). *Thromb Haemost*, 2004, vol. 92, 1066-1075

- **SHEVCHENKO, A.** ; **LOBODA, A.** ; **ENS, W.** ; **SCHRAVEN, B.** ; **STANDING, K. G.** ; **SHEVCHENKO, A.** Archived polyacrylamide gels as a resource for proteome characterization by mass spectrometry. *Electrophoresis*, 2001, vol. 22, 1194-1203

- *Disseminated Intravascular Coagulation | NHLBI, NIH*, 20 December 2017, www.nhlbi.nih.gov

- **LEVI M** ; **SCULLY M.** How I treat Disseminated Intravascular coagulation. *Blood*, 2018, vol. 131 (8), 845-854

- **PODOLSKA MJ et al.** *Autoimmunity 2018 - Review*

- *Cancer Immunol Immunother*, 2020

- *Breast Cancer Res*, 2019

- Neutrophil Extracellular Traps Associate With Clinical Stages in Breast Cancer Science Translational Medicine. *Pathol Oncol Res*, 19 October 2016, vol. 8 (361), 361-138

- **M STEN-LINDER** ; **C LINDER** ; **H STRANDER** ; **E MUNCK-WIKLAND** ; **P WERSÄLL** ; **S LINDER** ; **B WIMAN**. Angiostatin Fragments in Urine From Patients With Malignant Disease. *Anticancer Res*, 1999, vol. 19, 3409-14

- **BARBOSA DA CRUZ D et al.** DNA-bound Elastase of Neutrophil Extracellular Traps Degrades Plasminogen, Reduces Plasmin Formation, and Decreases Fibrinolysis: Proof of Concept in Septic Shock Plasma. *FASEB J*, vol. 20189 (33), 14270

- **OKAMOTO A et al.** Population based distribution of plasminogen.... *J Thromb Haemost*, 2003, vol. 1, 2397